# EUROPÄISCHE PATENTSCHRIFT

(11) **EP 3 723 576 B1**
(45) Veröffentlichungstag und Bekanntmachung des Hinweises auf die Patenterteilung: **23.02.2022**
(21) Anmeldenummer: 19728861.6
(22) Anmeldetag: 26.04.2019
(51) Int. Cl.: A61B 1/00, A61B 1/012, A61B 1/015

(54) **VORRICHTUNG UND VERFAHREN ZUR KONTAMINATIONSFREIEN DURCHFÜHRUNG EINER ENDOSKOPISCHEN UNTERSUCHUNG**
DEVICE AND METHOD FOR PERFORMING AN ENDOSCOPIC EXAMINATION FREE FROM CONTAMINATION
DISPOSITIF ET PROCÉDÉ DE RÉALISATION D'UN EXAMEN ENDOSCOPIQUE SANS CONTAMINATION

(30) Priorität: 27.04.2018 DE 102018110228
(43) Veröffentlichungstag der Anmeldung: 21.10.2020
(73) Patentinhaber: Kress, Jürgen, 84051 Essenbach (DE)
(72) Erfinder: Kress, Jürgen, 84051 Essenbach (DE)
(74) Vertreter: Pöhner, Wilfried Anton
(86) Internationale Anmeldenummer: PCT/DE2019/100380
(87) Internationale Veröffentlichungsnummer: WO 2019/206381

(56) Entgegenhaltungen:
- EP-A1- 1 578 255
- EP-B1- 1 578 255
- WO-A1-2009/086396
- US-A- 4 907 395
- US-A- 5 217 001
- US-A- 5 433 221
- US-B2- 8 241 208

## Beschreibung

Die vorliegende Erfindung betrifft ein Endoskop mit Kontaminationsschutzvorrichtung Die medizinische Endoskopie ist ein diagnostisches Verfahren zur Untersuchung von Körperöffnungen oder -höhlen, bei dem auch die Möglichkeit zur Durchführung minimal invasiver operativer Eingriffe gegeben ist. Das Verfahren beruht darauf eine bildgebende Sonde in die zu untersuchende Körperöffnung einzuführen.

Zu diesem Zweck werden Endoskope verwendet, welche aus einem Griffstück und einem daran anschließenden, starren oder flexiblen Schaft bestehen, an dessen Spitze Vorrichtungen zur Beleuchtung und Bildgebung vorgehalten sind. Zur Beleuchtung wird entweder das Licht einer außerhalb des Endoskops angeordneten Lichtquelle verwendet, das durch im Inneren des Endoskopschafts verlaufende Glasfaserbündel geleitet wird, oder, bei heutigen Endoskopen üblicher, durch in die Schaftspitze integrierte Lichtquellen, insbesondere LEDs, erzeugtes Licht eingesetzt. Zur Darstellung des Untersuchungsobjekts ist an der Schaftspitze eine Optik angeordnet, deren Bild bei Endoskopen modernster Bauart von einem Videochip registriert, weiterverarbeitet und über eine ebenfalls im Schaft verlaufende Datenleitung ausgelesen wird. Zur Manipulation des Untersuchungsobjektes, insbesondere zum dosierten Aufblähen von Hohlorganen und Körperhöhlen, ist an der Schaftspitze ferner eine Luftdüse vorgehalten, welche über einen im Inneren des Schafts verlaufenden Kanal von außerhalb des Endoskop angeordneten Pumpen oder Irrigatoren beaufschlagt werden kann.

Des Weiteren befindet sich an der Schaftspitze eine abgewinkelt angeordnete Wasserdüse, welche während der Untersuchung zum Abspülen von Körpersekreten von der Schaftspitze verwendet wird, insbesondere um die bildgebende Optik zu reinigen. Auch die Wasserdüse wird über einen im Schaftinneren verlaufenden Wasserkanal versorgt.

Außerdem weisen Endoskope üblicherweise einen durch den Schaft verlaufenden Instrumentierkanal auf, durch welchen mikromechanische Geräte wie Zangen, Greifer oder Schlingen an Bowdenzügen in den Untersuchungsraum geführt werden können, um bspw. eine Biopsie vorzunehmen.

Zusätzlich kann der Instrumentierkanal auch zur Absaugung verwendet werden. Dazu mündet er an seinem proximalen Ende im Bereich des Griffstücks in einem y-förmigen Verteilerstück, welches über seine beiden proximalen Schenkel sowohl Zugänglichkeit für ein Instrument als auch eine Anschlussmöglichkeit für eine Pumpe bietet.

Zur Bedienung durch den untersuchenden Arzt sind an dem Griffstück Ventile für Luft, Wasser und Absaugung vorgehalten sowie Bedienelemente zum Abwinkeln und Feinpositionieren der Schaftspitze, welche über im Schaft verlaufende Bowdenzüge angesteuert wird. Die Grobpositionierung des Schaftes in der zur untersuchenden Körperöffnung wird durch händisches Vorschieben oder Zurückziehen vorgenommen. Die Bedienung der Instrumentiermechanik erfolgt über ein separates, am proximalen Ende des Instruments vorgehaltenes Bedienelement.

In der Praxis stellen die bei endoskopischen Untersuchungen auftretenden Kontaminationen mit Körperflüssigkeiten ein schwerwiegendes Problem dar. Solche Kontaminationen betreffen dabei so-wohl das Endoskop, die Untersuchungsumgebung, das medizinische Personal als auch, bei Verwendung eines nicht ausreichend sterilisierten Endoskops, den untersuchten Patienten selbst.

Der Aufwand zur Reinigung und Sterilisation eines gebrauchten Endoskops ist beträchtlich: Er umfasst zahlreiche Arbeitsschritte des mechanischen Reinigens, des Spülens, der Anwendung von Enzymreinigern, des Desinfizierens und des Trocknens, und nimmt in der Praxis typischerweise einen Zeitaufwand von 45 Minuten in Anspruch. Diese Prozedur bedingt hohe Personal- und Gerätekosten und stellt einen entscheidenden Faktor in der Amortisationsdauer der in der Anschaffung kostspieligen Endoskope dar. Es findet dadurch ferner eine unerwünschte Kapitalbindung statt, da für die Gewährleistung eines kontinuierlichen Untersuchungsbetriebes eine Vielzahl an Endoskopen vorgehalten werden muss.

Neben diesen ökonomischen Aspekten stellt insbesondere das Kontaminationsrisiko für das die Endoskopie durchführende, medizinische Personal ein schwerwiegendes Problem dar. So kommt der behandelnde Arzt durch das händische Verschieben des Endoskopschafts im Zuge der Untersuchung unweigerlich mit Körperflüssigkeiten in Kontakt. Auch bei der Instrumentenentnahme aus dem verschmutzten Instrumentierkanal ist das Kontaminationsrisiko evident. Ebenso ist die im Zuge der Untersuchung entstehende Verschmutzung der Untersuchungsumgebung, insbesondere der Patientenliege, zu beachten.

Im Stand der Technik sind Kontaminationsschutzvorrichtungen für medizinische Endoskope bekannt. So beschreibt die Patentschrift EP1578255B1 eine Schutzhülle aus einem elastischen Material, welche ausgehend von seiner Spitze kondomartig über den Endoskopschaft gezogen wird. Diese Hülle besteht dabei zumindest im Bereich der Endoskopoptik aus einem optisch transparenten Material, um die endoskopische Bildgebung nicht zu beeinträchtigen. Da bei Verwendung einer solchen Schutzhülle die im Inneren des Endoskopschafts verlaufenden Arbeitskanäle nicht mehr zugänglich sind, lehrt die Patentschrift separate Luft-, Wasser- und Instrumentierkanäle vorzuhalten, die zwischen Endoskopschaft und Schutzhülle anzuordnen sind und am distalen Ende luft-, wasser- und keimdicht mit der Schutzhülle verbunden sind. Bei Verwendung einer solchen Schutzvorrichtung nach dem Stand der Technik lässt sich eine direkte Kontamination des Endoskops während der Untersuchung vermeiden.

Ungelöst bleibt jedoch das Problem der Kontamination des medizinischen Personals und der Untersuchungsumgebung, woraus in weiterer Folge auch eine indirekte Kontamination des Endoskops resultieren kann. Nachteilig ist außerdem, dass durch Verwendung eines separaten, außenliegenden Instrumentierkanals, welcher üblicherweise einen nicht unbeträchtlichen Durchmesser aufweist, eine signifikante Vergrößerung des Gesamtquerschnitts des Endoskops einhergeht, wodurch die endoskopische Untersuchung für den Patienten mit erhöhten Schmerzen verbunden sein kann bzw. bestimmte kleinräumige Untersuchungsbereiche gar nicht mehr zugänglich sind.

Die vorliegende Erfindung möchte vor diesem Hintergrund die Aufgabe lösen, einen verlässlichen und umfassenden Kontaminationsschutz für endoskopische Untersuchung zu gewährleisten, welcher Verschmutzung des Endoskops, der Untersuchungsumgebung und des medizinischen Personals drastisch reduziert oder vermeidet, dabei aber zu keinerlei Einschränkung von Handhabbarkeit und Verwendbarkeit des Endoskops führt.

Erreicht wird dies durch ein Endoskop mit Kontaminationsschutzvorrichtung gemäß einem der Ansprüche 1 - 13, welche gemäß einem der Ansprüche 14-18 angebracht werden kann.

Die wesentliche Idee der vorliegenden Erfindung besteht dabei darin, zwischen der zu untersuchenden Körperöffnung des Patienten und dem proximalen Ende des eingeführten Endoskopschafts ein Schutzvolumen vorzuhalten, welches alle im Zuge der Untersuchung freiwerdenden Körperflüssigkeiten aufsammelt und von Kontakt mit der Umgebung fernhält. Dieses Volumen wird begrenzt durch eine Innenhülle, welche im Wesentlichen der im Stand der Technik beschriebenen Schutzhülle auf dem Endoskopschaft entspricht, sowie durch eine größer dimensionierte Außenhülle, die am proximalen Ende der Innenhülle an selbiger luft-, wasser- und keimdicht befestigt ist. Während der endoskopischen Untersuchung wird lediglich die Innenhülle in die zu untersuchende Körperöffnung eingeführt, so dass alle beim Herausziehen des Endoskopschafts an der Innenhülle anhaftenden Körperflüssigkeiten oder sonstige Verschmutzungen von der Außenhülle schützend bedeckt werden.

Im Weiteren werden vorteilhafte Ausführungen des erfindungsgemäßen Endoskops mit Kontaminationsvorrichtung vorgestellt, welche in geeigneter Form miteinander kombiniert werden können.

Um das erfindungsgemäß durch Außen- und Innenhülle aufgespannte Schutzvolumen vollständig von der Untersuchungsumgebung abzuschließen, sind am distalen Ende der Außenhülle geeignete Mittel vorzuhalten. Diese bestehen aus Anschlussstücken, welche mit patientenseitig vorgehaltenen, erfindungsgemäßen Vorrichtungen verbunden werden können.

Für koloskopische Untersuchung des Darmes wird eine solche Vorrichtung durch eine spezielle Windelhose für den zu untersuchenden Patienten dargestellt, die eine Öffnung mit einem Aufnahmeteil aufweist, an welches das Anschlussstück der Außenhülle luft-, wasser- und keimdicht angeschlossen werden kann. Auf diese Weise wird das Schutzvolumen um einen Abschnitt der Windelhose erweitert und bei passgenauem, dichtem Sitz der Windelhose am Patienten von der Untersuchungsumgebung im Wesentlichen abgeschlossen.

In Analogie dazu kann bei gastroskopischen Untersuchungen der Speiseröhre oder des Magens ein geeigneter Beißring für die Aufnahme des Anschlussstückes der Außenhülle verwendet werden, an dem ein erfindungsgemäßes Anschlussteil angeformt ist. Um eine Kontamination der Außenseite der Außenhülle durch aus den Mundwinkeln des Patienten austretende Körperflüssigkeiten zu vermeiden, kann ein solcher Beißring maskenartig um die Kieferpartie erweitert werden, wobei dafür Sorge zu tragen ist, dass damit keine signifikante Beeinträchtigung der Atemtätigkeit des Patienten einhergeht.

Alternativ kann eine geeignete dimensionierte Trennwand zwischen den zu Patienten und das die Untersuchung durchführende, medizinische Personal positioniert werden. An dieser ist erfindungsgemäß eine relativ zur Patientenliege passend angeordnete Öffnung vorzuhalten, die mit einem Aufnahmeteil zur Verbindung mit dem Anschlussstück der Außenhülle versehen ist. Mit einem solchen Aufbau wird zwar die unmittelbare Untersuchungsumgebung, insbesondere die Patientenliege, Verschmutzungen während der Untersuchung ausgesetzt, nicht aber das auf der anderen Seite der Trennwand befindliche medizinische Personal. Durch geeignete Ausgestaltung der Trennwand, insbesondere in Form einer Kabine, kann das kontaminierte Volumen weiter eingeschränkt bzw. ganz abgeschlossen werden.

Die Verbindung von Anschlussteil und -stück soll in allen vorgenannten Ausführungsformen luft-, wasser- und keimdicht ausgeführt sein und gesichert gegen unbeabsichtigtes Öffnen während der Durchführung der Endoskopie. Zu diesem Zweck sind geeignete Schraub-, Steck-, Klemm- oder Magnetverbindungen bekannt.

Als zusätzliche Schutzmaßnahme kann die Außenhülle noch über das proximale Ende der Innenhülle hinausgeführt werden und auch das Griffstück des Endoskops samt Griffaufsatz schützend umhüllen.

Innen- und Außenhülle sollte aus einem für medizinische Zwecke üblicherweise verwendeten Material bestehen, welches luft- und wasserdicht ist sowie undurchlässig für Krankheitskeime. Es muss ferner ein ausreichendes Maß an Widerstandsfähigkeit gegen mechanischen Verschleiß wie Reißen oder Abrasion aufweisen, sowie chemische Beständigkeit gegen im Zuge von endoskopischen Untersuchungen anzutreffende Substanzen, bspw. Säuren oder Fette. Zum Zwecke der Montage sollten Innen- und Außenhülle insbesondere leicht aufrollbar sein. Als geeignete polymere Werkstoff kommen bspw. Polybuten (PB) oder Cycloolefin-Polymere (COP) in Frage.

Zum Kontaminationsschutz des im Inneren des Endoskopschafts verlaufenden Instrumentierkanals lehrt vorliegende Erfindung in vorteilhafter Ausführung die Verwendung eines Arbeitsschlauchs, der den Instrumentierkanal auf seiner ganzen Länge auskleidet und hinter dem proximalen Ende des Schaftes aus der Instrumentierkanalöffnung herausragt, die bei handelsüblichen Endoskopen ohne Kontaminationsschutz zum Einführen der Instrumente dient, d.h. nicht der Absaugung. Am distalen Ende der Innenhülle mündet der Arbeitsschlauch stirnseitig in einer Arbeitsschlauchöffnung, an die er nahtlos angeformt ist.

Um den Arbeitsschlauch sowohl zur Gerätedurchführung als auch zur Absaugung verwenden zu können, mündet er erfindungsgemäß hinter der proximalen Instrumentierkanalöffnung in einem y-förmigen Zwei-Wege-Schlauchverteiler, der in eine passende Aufnahme am Halter eines Griffaufsatzes eingesteckt oder alternativ direkt an der proximalen Instrumentierkanalöffnung befestigt ist.

Der Griffaufsatz der ersten Variante kann weiterhin Befestigungen für die Ventile, also das Luftventil, Wasserventil und das Absaugventil umfassen. In der zweiten Variante wird vorgeschlagen, den Schlauchverteiler mittels einer axial verschiebbar aufgesetzten Ringmutter auszustatten, mit welcher der Schlauchverteiler lösbar an dem üblicherweise vorhandenen Luerlock-Anschluss des proximalen Instrumentierkanalendes befestigbar ist.

Der Arbeitsschlauch mündet im distalen Ende dieses Schlauchverteilers, so dass er durch einen der beiden proximalen Schenkel des Schlauchverteilers für ein Instrument und durch den anderen zur Absaugung zugänglich ist.

Die Absaugung wird dabei in der ersten Variante über ein ebenfalls am Halter des Griffaufsatzes vorgehaltenes Saugventil kontrolliert, das über einen Saugschlauch mit einem proximalen Ende des Zwei-Wege-Schlauchverteilers verbunden ist.

Für Endoskope mit mehr als einem Instrumentierkanal, bekannt sind beispielsweise Endoskope mit zwei Instrumentierkanälen, kann der erfindungsgemäße Kontaminationsschutz auch mehr als einen Arbeitsschlauch, insbesondere jeweils einen Arbeitsschlauch pro Instrumentierkanal des Endeskops, aufweisen.

Ebenfalls können mehr als ein Wasser- oder Luftschlauch vorhanden sein. Es sind beispielsweise Endoskope mit einem zusätzlichen Wasserkanal bekannt, auch als Jet-Kanal bezeichnet, welcher in der Endoskopspitze frei mündet und nicht zur Spülung der Optik gedacht ist, sondern dazu, die Operations- oder Untersuchungsstelle mit Wasser zu spülen. Für solche Endoskope kann der erfindungsgemäße Kontaminationsschutz um einen weiteren Wasserschlauch samt Ventil ergänzt werden. Alternativ kann der Wasserschlauch auch doppellumig sein, wobei ein Lumen zur Spülung der Optik und das andere als Jet-Kanal verwendet wird.

Auf diese Weise wird ein inwendiger Kontaminationsschutz des Endoskopschafts realisiert, welcher eine uneingeschränkte Verwendung des Instrumentierkanals zur Durchführung mikromechanischer Geräte und zur Absaugung ermöglicht.

In einer weiteren Ausführungsform, bei der die Außenhülle als redundante Schutzmaßnahme hinter dem proximalen Schaftende weitergeführt wird und Griffstück und -aufsatz umhüllt, ist der Zwei-Wege-Schlauchverteiler in die Außenhülle integriert, also etwa luft-, wasser- und keimdicht daran angeschweißt.

In einer weiteren vorteilhaften Ausführungsform ist an dem zum Einführen eines Instrumentes vorgesehenen, proximalen Ende des Zwei-Wege-Schlauchverteilers ein erfindungsgemäßer Instrumentenschutz angeschlossen. Dieser dient dazu, das nach Abschluss des Instrumentiervorgangs aus dem Arbeitskanal her-ausgezogene Instrument samt zugehöriger Bowdenzüge und etwaig entnommener Gewebeprobe in einen schützenden Instrumentenschlauch einzuziehen und somit medizinisches Personal und Untersuchungsumgebung vor Kontakt mit anhaftenden Verschmutzungen zu bewahren. Die Länge dieses Instrumentenschlauchs ist folglich größer als die Länge des Arbeitsschlauches zu dimensionieren, so dass der gesamte verschmutzte Abschnitt des Instruments und der Bowdenzüge darin Platz findet. Die beiden Enden des Instrumentenschlauchs sind mit je einer Kappe mit stirnseitiger Öffnung versehen, welche der Verbindung mit dem Zwei-Wege-Schlauchverteiler bzw. einem Griffstücks des Instruments dienen. Der Instrumentenschlauch sollte aus einem balgartig zusammenschiebbarem Material bestehen, so dass sich der gesamte Instrumentenschutz auf ein kleines Maß zusammen-drücken lässt, was eine übersichtliche und kompakte Anbringung am Griffansatz gewährleistet.

Zur Befestigung der Innenhülle an der Spitze des Endoskopschafts lehrt vorliegende Erfindung den distalen Endabschnitt der Innenhülle vorteilhaft in Form einer steifen Kappe auszuführen, die auf einen Klemmbügel gesteckt wird, welcher die Mantelfläche des Endoskopschafts umfasst.

Die Steckverbindung kommt dadurch zustande, dass eine den Klemmbügel umlaufende Feder in eine entsprechende Nut an der Innenseite der Kappe einrastet. Um die Kappe einfach und schnell wieder lösen zu können, kann an ihr ein sich über einen Teil des Schaftumfangs erstreckender bügelartiger Klemmer angeformt sein, mittels dessen bei entsprechender Kraftausübung der Umfang der offenen Stirnseite der elastischen Kappe soweit erweitert werden kann, dass sie sich wieder von der Feder des Klemmbügels lösen und von der Schaftspitze abziehen lässt.

Alternativ kann die Kappe auch ohne Verwendung eines Klemmbügels mit Hilfe an der offenen Stirnseite der Kappe angeformte, axial verlaufende elastische Branchen auf die Schaftspitze geklemmt werden.

Zur platzsparenden Durchführung von Luft- und Wasserschläuchen sollte die Feder entsprechende Aussparungen aufweisen, und die Schläuche in vorteilhafter Ausführungsform in das Kappeninnere geführt werden und in Öffnungen auf der Stirnfläche münden.

Der die Kappe fixierende Klemmbügel sollte von einer Dichtung unterlegt sein, die bspw. aus Silikon, Teflon, EPDM Kautschuk oder einem selbstklebenden Adhäsiv bestehen kann.

In weiterer vorteilhafter Ausführung sind Innen- und Außenhülle am proximalen Ende des Endoskopschafts befestigt. Dazu kann ein weiterer von einer Dichtung unterlegter Klemmbügel dienen, auf welchem Innen- und Außenhülle aufliegen und von einer umlaufenden Klemme fixiert werden.

Weder der proximale noch der distale Klemmbügel müssen zwischen zwei kontaminationsgeschützten Untersuchungen abgenommen werden, wodurch die zur Vorbereitung des Endoskops für eine weitere Untersuchung benötigte Zeit vorteilhaft verkürzt wird.

Es ist ferner vorteilhaft Innen - und Außenhülle derart einteilig zu fertigen, dass sie am proximalen Ende der Innenhülle miteinander verbunden sind. Eine solche Verbindung kann in der Fertigung bspw. durch ein Schweißverfahren realisiert werden, etwa das zur Verbindung von polymeren Werkstoffen geeignete Ultraschall-Schweißen. Dadurch wird eine potentielle Schwachstelle bzgl. der Dichtheit der Schutzvorrichtung eliminiert und die Anbringung der Hüllen auf dem Endoskopschaft erleichtert. Zur handlicheren Anbringung bzw. Demontage der Kontaminationsschutzvorrichtung kann die Innenhülle entlang des Endoskopschafts vorteilhaft von einem flexiblen Gewebeschlauch unterlegt werden.

Die zu Anbringung und Demontage der erfindungsgemäßen Kontaminationsschutzvorrichtung an ein Endoskop notwendigen Schritte werden nachstehend im Rahmen der Figurenbeschreibung detailliert angegeben.

Bei der Verwendung eines erfindungsgemäßen Endoskops ist es vorteilhaft, die Kontaminationsschutzvorrichtung auf Dichtheit zu überprüfen. Eine solche Dichtheitsprüfung kann zum einen nach erfolgter Anbringung der Vorrichtung am Endoskop erfolgen, um ggfs. Material- und Montagefehler zu entdecken.

Ein geeignetes Verfahren der Dichtheitsprüfung wäre bspw. die Differenzdruck-Methode. Das distale Ende der Außenhülle mit dem ggfs. daran angeformten Anschlussstück ist an das Prüfgerät anzuschließen, so dass die Dichtheitsprüfung dem zwischen Innen- und Außenhülle aufgespannten Schutzvolumen gilt. Dazu ist es außerdem notwendig Luft- und Wasserschlauch abzudichten, bspw. durch geeignete Stöpsel.

Bei Verwendung einer Ausführungsform der Kontaminationsschutzvorrichtung mit Arbeitsschlauch sollte ein solcher Abdichtstöpsel in das proximale Schlauchende eingesteckt werden, so dass im Zuge der Dichtheitsprüfung auch der vom Arbeitsschlauch ausgekleidete Instrumentierkanal im Endoskopschaft überprüft wird.

Es ist ferner vorteilhaft, auch nach Abschluss der endoskopischen Untersuchung eine entsprechende Dichtheitsprüfung vorzunehmen. Dadurch kann überprüft werden, ob das Endoskop im Zuge der Untersuchung kontaminiert worden ist und entsprechende Reinigungsmaßnahmen vor der nächsten Verwendung durchzuführen sind. Vor der Dichtheitsprüfung sind die Außenhülle an ihrem distalen Ende sowie Luft-, Wasser- und ggfs. Arbeitsschlauch im Bereich des Griffstücks derart zu durchtrennen, dass die abgetrennten Enden luftdicht verschlossen sind. Dies kann bspw. mittels einer Brennschere geschehen.

Bei Verwendung einer Ausführungsform der Kontaminationsschutzvorrichtung mit einteilig gefertigter Innen- und Außenhülle kann die an die Untersuchung anschließende Dichtheitsprüfung auch an der bereits demontierten Kontaminationsschutzvorrichtung vorgenommen werden.

Als Zugang für das Dichtheitsprüfgerät zum kontaminierten Volumen der Kontaminationsschutzvorrichtung sollte in vorteilhafter Ausführungsform das proximale Ende des Arbeitsschlauchs verwendet werden, welches zu diesem Zwecke neuerlich geöffnet werden muss. Ist in einer einfacheren Ausführungsform der Kontaminationsschutzvorrichtung kein Arbeitsschlauch vorhanden, so kann als Zugang stattdessen eine neuerliche Öffnung in die Außenhülle geschnitten werden.

Weitere Eigenschaften, Merkmale und Vorteile vorliegender Erfindung ergeben sich aus der im Folgenden anhand der Figuren näher erläuterten, beispielhaften Ausführungen. Diese sollen vorliegende Erfindung nur illustrieren und in keiner Weise einschränken.

Es zeigen:
- Figur 1:: Gesamtansicht eines erfindungsgemäßen Endoskops
- Figur 2:: Detailansicht der Spitze eines erfindungsgemäßen Endoskops
- Figur 3a-d:: Anbringung des distalen Klemmbügels
- Figur 4a,b:: Anbringung des distalen Endes der Innenhülle
- Figur 5a-c:: Anbringung der Kontaminationsschutzvorrichtung am proximalen Schaftende
- Figur 6a-c:: Anbringung der Kontaminationsschutzvorrichtung am Griffstück
- Figur 7:: Vorteilhafte Ausführungsform zur bedienerfreundlichen Anbringung der Schutzvorrichtung an den Schaft
- Figur 8:: Vorteilhafte Ausführungsform einer Kappe mit Klickverschluss
die endoskopische Untersuchung für den Patienten mit erhöhten Schmerzen verbunden sein kann bzw. bestimmte kleinräumige Untersuchungsbereiche gar nicht mehr zugänglich sind.

In der Patentschrift US 4,907,395 wird ein System zum Packen von Wegwerf-Endoskophüllen vorgeschlagen, welches sicherstellt, dass die Endoskophüllen während dem Transport, der Lagerung und dem Aufziehen auf ein Endoskop nicht kontaminiert werden und weiterhin auch die Verbreitung von der benutzten Hülle ausgehender Kontamination unterbindet.

Die internationale Veröffentlichungsschrift WO 2009/086396 A1 beschreibt eine Hülle für ein Endoskop mit einer Kappe, welche auf das distale Ende des Endoskopschaftes aufsetzbar ist, und einem an der Kappe befestigten aufgerollten, flexiblen, länglichen Schlauch, welche nach dem Aufsetzen der Kappe entlang des Endoskopschaftes abrollbar ist.

Die vorliegende Erfindung möchte vor diesem Hintergrund die Aufgabe lösen, einen verlässlichen und umfassenden Kontaminationsschutz für endoskopische Untersuchung zu gewährleisten, welcher Verschmutzung des Endoskops, der Untersuchungsumgebung und des medizinischen Personals drastisch reduziert oder vermeidet, dabei aber zu keinerlei Einschränkung von Handhabbarkeit und Verwendbarkeit des Endoskops führt.

Erreicht wird dies durch ein Endoskop mit Kontaminationsschutzvorrichtung gemäß einem der Ansprüche 1 - 13, welche gemäß einem der Ansprüche 14 - 18 angebracht werden kann.

Die wesentliche Idee der vorliegenden Erfindung besteht dabei darin, zwischen der zu untersuchenden Körperöffnung des Patienten und dem proximalen Ende des eingeführten Endoskopschafts ein Schutzvolumen vorzuhalten, welches alle im Zuge der Untersuchung freiwerdenden Körperflüssigkeiten aufsammelt und von Kontakt mit der Umgebung fernhält. Dieses Volumen wird begrenzt durch eine Innenhülle, welche im Wesentlichen der im Stand der Technik beschriebenen Schutzhülle auf dem Endoskopschaft entspricht, sowie durch eine größer dimensionierte Außenhülle, die am proximalen Ende der Innenhülle an selbiger luft-, wasser- und keimdicht befestigt ist. Während der endoskopischen Untersuchung wird lediglich die Innenhülle in die zu untersuchende Körperöffnung eingeführt, so dass alle beim Herausziehen des Endo-skopschafts an der Innenhülle anhaftenden Körperflüssigkeiten oder sonstige Verschmutzungen von der Außenhülle schützend bedeckt werden.

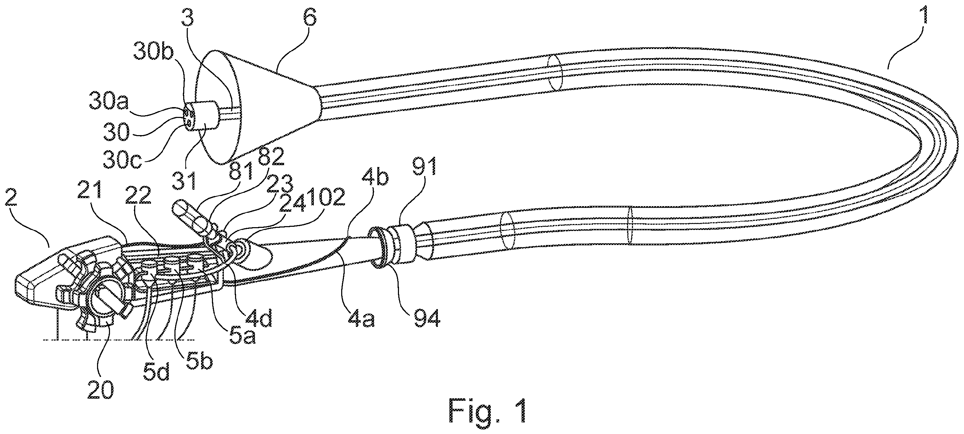

Im Weiteren werden vorteilhafte Ausführungen des erfindungsgemäßen Endoskops mit Kontaminationsvorrichtung vorgestellt, welche in geeigneter Form miteinander kombiniert werden können.
in die zu untersuchende Körperöffnung eines Patienten eingeführten Teils des Endoskops gewährleistet, die dadurch vervollständigt wird, dass der im Inneren des Endoskopschafts 1 verlaufende Instrumentierkanal 10 von dem an die Arbeitskanalöffnung 30c anschließenden Arbeitskanal ausgekleidet ist. Der so versiegelte Endoskopschaft wird von der Außenhülle 6 umhüllt, sodass der Zwischenraum ein Schutzvolumen bildet, in dem während der endoskopischen Untersuchung aus der Körperöffnung des Patienten austretende Körperflüssigkeiten aufgefangen werden. Patientenseitig wird dieses Schutzvolumen abgedichtet durch das an der Außenhülle 6 vorgehaltene Anschlussstück 61, welches beispielsweise an eine geeignete Windelhose 7a oder einen Beißring 7b angeschlossen werden kann. Die Abdichtung des Schutzvolumens am proximalen Ende des Endoskopschafts 1 wird in der in Fig. 1 dargestellten Ausführungsform durch die Klemme 91 gewährleistet, welche Innen- und Außenhülle 3, 6 auf einen darunter befindlichen proximalen Klemmbügel 93 presst. Die darüber hinaus stehenden Enden von Innen- und Außenhülle 3, 6 werden in die Klammer 94 aufgenommen und somit bspw. gegen Einreißen gesichert. Zur Durchführung von Luft- und Wasserschläuchen 4a, 4b über das proximale Ende des Schutzvolumens hinaus sind in dem unter Klemme 91, Außen- und Innenhülle 6, 3 befindlichen proximalen Klemmbügel 93 entsprechende Schlitze vorgehalten. An das Griffstück 2 des Endoskops ist der Griffaufsatz 21 mit dem Ventilhalter 22 und dem Instrumentierhalter 23 aufgesetzt, in welche Luft-Wasser- und Saugventil 5a, 5b, 5d sowie der Zwei-Wege-Schlauchverteiler 24 eingesteckt sind. Der Zwei-Wege-Schlauchverteiler 24, der mit seinem distalen Ende an den aus der proximalen Instrumentierkanalöffnung 102 herausragenden Arbeitsschlauch 4c angeschlossen ist, ermöglicht über seine beiden proximalen Enden Zugang zum Instrumentierkanal 10. Der proximale Schenkel des Schlauchverteilers 24, welcher die geradlinige Verlängerung des distalen Schenkels bildet, ist für ein an einem Bowdenzug durchgeführtes Instrument 80 vorgesehen, mittels dem minimal invasive operative Eingriffe und Biopsien durchgeführt werden können. An den abgewinkelten Schenkel des Schlauchverteilers 24 sind der Saugschlauch 4d und das zugehörige Saugventil 5d angeschlossen. Luft- und Wasserventil 5a, 5b sind mit den zum distalen Ende des Endoskopschafts führenden Luft- und Wasserschläuchen 4a, 4b verbunden. Die aus den Ventilen 5a, 5b, 5d vom Endoskop weglaufenden Schläuche sind mit geeigneten, im Rahmen vorliegender Erfindung nicht beanspruchten Vorrichtungen zur Beaufschlagung mit Unter- oder Überdruck oder Wasser verbunden.

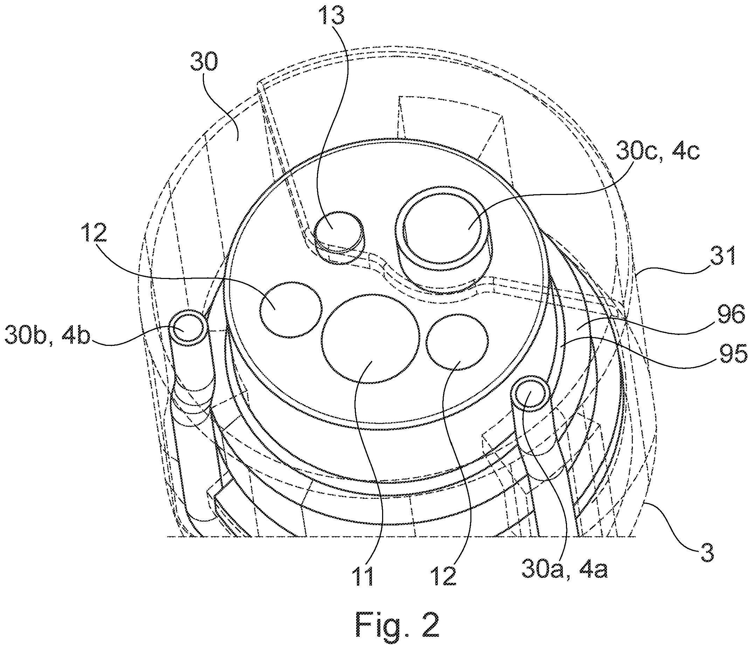

**Fig. 2** zeigt eine Detailansicht des distalen Endes einer vorteilhaften Ausführungsform des erfindungsgemäßen Endoskops. Unter der optisch transparenten Stirnseite 30 der Innenhülle 3 bzw. der Kappe 31 befinden sich die Beleuchtungsvorrichtungen 12, die zur Bildgebung dienende Optik 11 sowie die Düse 13, die bei erfindungsgemäßer Verwendung des Endoskops nicht betrieben wird. Die Stirnseite 30 wird durchstoßen von dem Luftschlauch 4a, welcher in der Luftschlauchöffnung 30a mündet, dem Wasserschlauch 4b mit der Wasserschlauchöffnung 30b, sowie dem im Instrumentierkanal 10 verlaufenden Arbeitsschlauch 4c mit der Arbeitsschlauchöffnung 30c. Ferner ist in Fig. 2 der distale Klemmbügel 96 zu sehen, welcher von der distalen Dichtung 95 unterlegt die Mantelfläche des Endoskopschafts 1 umschließt und als Aufnahme für die darauf aufgesteckte Kappe 31 fungiert.

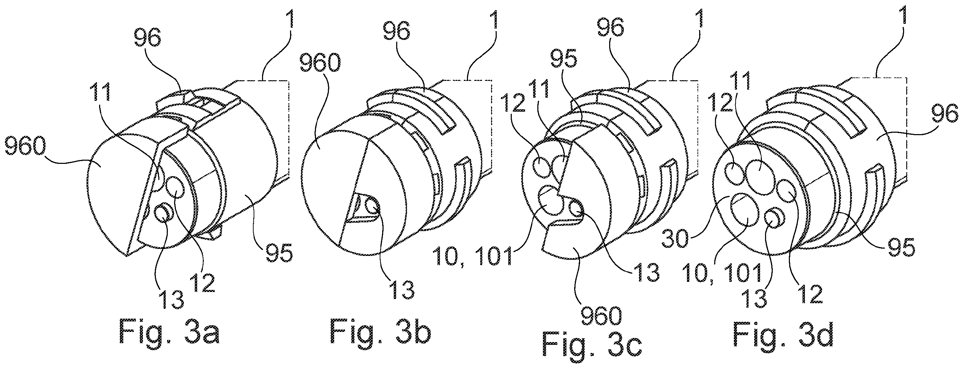

Anhand der Fig. 3 bis 6 sollen Details einer besonders vorteilhaften Ausführungsform des erfindungsgemäßen Endoskops dargestellt und die zur Anbringung der Kontaminationsschutzvorrichtung notwendigen Schritte illustriert werden.

Die **Fig. 3a** bis **3d** zeigen die Montage eines Klemmbügels 96 am distalen Ende des Endoskopschafts 1. Um einen korrekten Sitz der darauf anzubringenden Kappe 31 zu gewährleisten, sind bei der Montage des Klemmbügels 96 sowohl seine axiale als auch seine radiale Positionierung auf der Mantelfläche des Endoskopschafts 1 von Bedeutung. Es muss gewährleistet sein, dass die Stirnseite 30 der Kappe 31 derart von der Stirnseite des Endoskopschafts beabstandet ist, dass einerseits keine signifikante Einschränkung des Blickwinkels der Endoskopoptik 11 hervorgerufen wird, andererseits aber ausreichender Platz besteht um Luft- und Wasserschläuche 4a, 4b von der Außenseite des Endoskopschafts 1 an die Stirnseite 30 der Kappe 31 heranzuführen. Des Weiteren muss gewährleistet sein, dass die in vorteilhafter Ausführungsform auf der Stirnseite 30 der Kappe 31 angeordnete Arbeitsschlauchöffnung 30c in Deckung vor die Instrumentierkanalöffnung 101 an der Stirnseite des Endoskopschafts gesetzt wird. Ferner dürfen die Luft- und Wasserschlauchöffnungen 30a, 30b nicht die Beleuchtungsvorrichtungen 12 an der Schaftspitze bedecken oder abschatten. Vorliegende Erfindung lehrt daher die vorteilhafte Verwendung einer Positionierhilfe 960 für die Montage des Klemmbügels 96, wie in Fig. 3a bis 3d illustriert. Die Positionierhilfe 960 ist mit dem Klemmbügel 96 über Sollbruchstellen verbunden und wird nach erfolgter Montage abgebrochen. Die wesentlichen Elemente der Positionierhilfe 960 sind ein auf ihrer Stirnseite angeordneter und ins Innere weisender Zapfen, welcher in die Instrumentierkanalöffnung 101 eingesetzt wird, sowie eine stirnseitige Öffnung, welche über die aus der Schaftspitze herausragende Düse 13 gelegt wird. Dadurch ist die radiale Orientierung der Positionierhilfe samt daran befestigtem Klemmbügel eindeutig festgelegt, und es ist gewährleistet, dass die zur Durchführung von Luft- und Wasserschläuchen 4a, 4b vorgesehenen Aussparungen in der den Klemmbügel umlaufenden Feder im eingangs beschriebenen Sinne korrekt positioniert sind. Die axiale Position des Klemmbügels 96 wird dadurch festgelegt, dass die Positionierhilfe 960 formschlüssig an die Stirnfläche der Schaftspitze angedrückt wird.

Klemmbügel 96 und Positionierhilfe 960 sind vorteilhaft jeweils zweiteilig ausgeführt, und die Verbindung der beiden Klemmbügelhälften kommt durch ein klammerartiges Einrasten zustande.

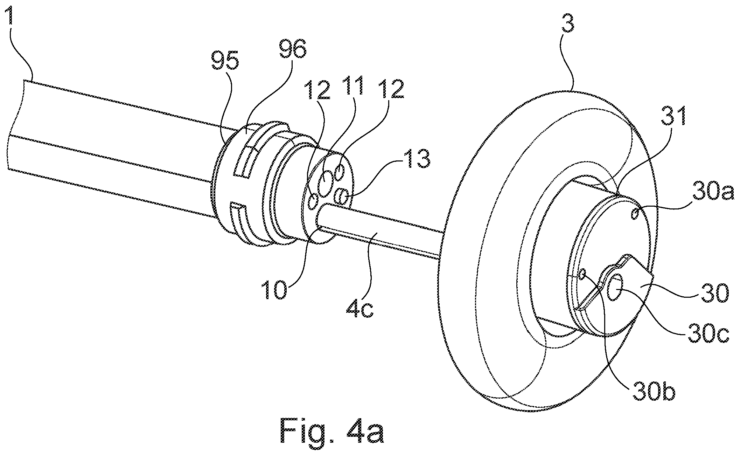

**Fig. 4a** zeigt eine Detailansicht des distalen Endes des Endoskopschafts 1 während der Montage einer Ausführungsform erfindungsgemäßer Kontaminationsschutzvorrichtung. Die Innenhülle 3 ist dabei zunächst bis an die Kappe 31 aufgerollt. Der Arbeitsschlauch 4c wird in den Instrumentierkanal 10 im Endoskopschaft 1 eingeführt und der hier nicht wiedergegebene Luft- und Wasserschlauch 4a, 4b werden an der Außenseite des Schaftes 1 verlegt. Die distale Dichtung 95 und der distale Klemmbügel 96 sind wie unter Fig. 3a bis 3d illustriert um den Endoskopschaft 1 montiert.

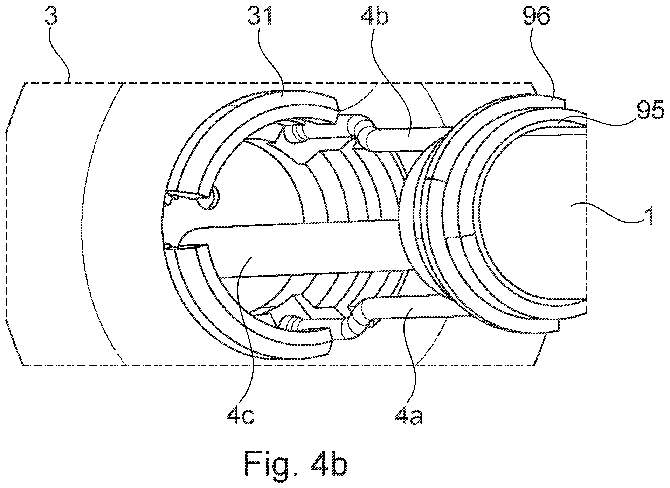

**Figur 4b** zeigt eine Ansicht derselben Montagesituation aus entgegengesetzter Perspektive. Darin ist die in die Kappe 31 eingeprägte Nut zu erkennen, in welche die den Klemmbügel 96 umlaufende Feder zum Zwecke der Verbindung einrastet.

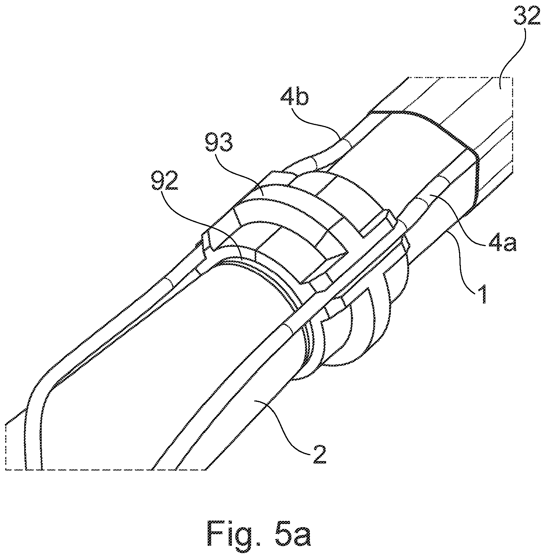

**Fig. 5a** illustriert die Anbringung einer vorteilhaften Ausführungsform erfindungsgemäßer Kontaminationsschutzvorrichtung am proximalen Ende des Endoskopschafts 1. Dargestellt sind der proximale Klemmbügel 93 auf der Dichtung 92 sowie die Luft- und Wasserschläuche 4a, 4b, für deren Durchführung entsprechende Aussparungen am Klemmbügel 93 vorgehalten sind. Am oberen Bildrand ist der Gewebeschlauch 32 dargestellt, welcher den Endoskopschaft 1 samt daran verlaufender Luft- und Wasserschläuche 4a, 4b fest umhüllt und eine Unterlage für die abzurollende Innenhülle 3 bildet.

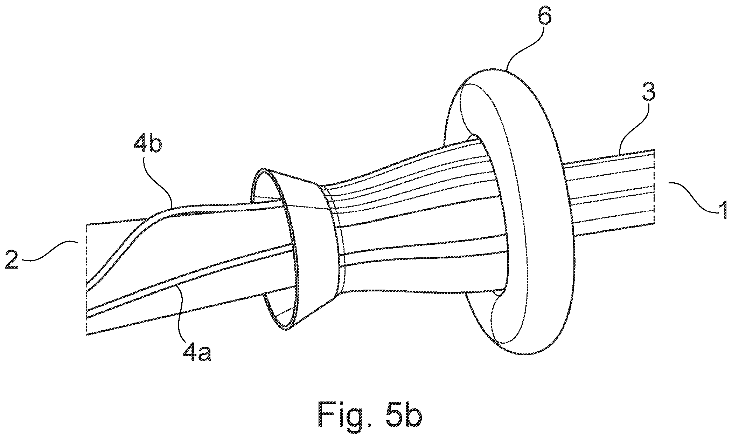

In **Fig. 5b** ist die Innenhülle 3 vollständig über den Endoskopschaft 1 abgerollt worden, derart, dass sie auch den proximalen Klemmbügel 93 überdeckt. Des Weiteren ist die Außenhülle 6 im aufgerollten Zustand über die Innenhülle 3 hochgeschoben worden, ebenfalls bis zum proximalen Klemmbügel 93. Bei Verwendung einer solchen Ausführungsform, bei der Innen- und Außenhülle 3, 6 einteilig gefertigt, d.h. etwa miteinander verschweißt sind, vereinfacht sich die Montage dadurch, dass die Außenhülle 6 nicht separat über die Innenhülle 3 bis zum proximalen Schaftende hochgeschoben werden muss, sondern dies bereits während des Abrollens der Innenhülle 3 geschieht.

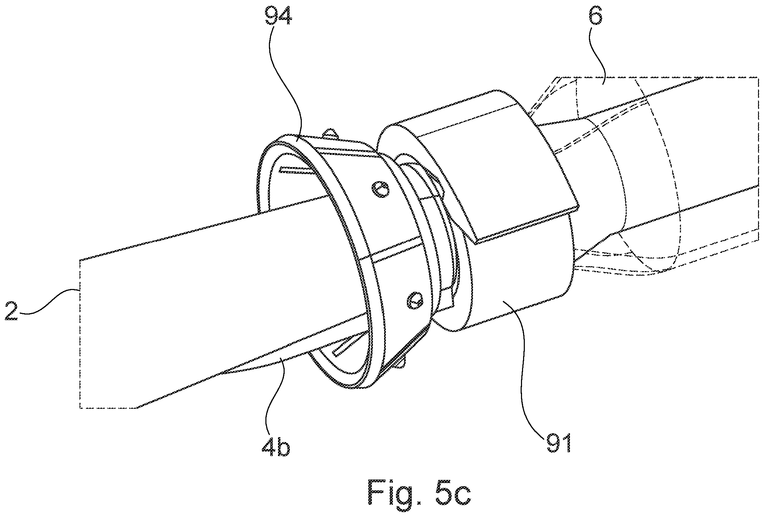

**Fig. 5c** zeigt die formschlüssige Fixierung von Innen- und Außenhülle 3, 6 mit Hilfe der Klemme 91, welche die beiden Hüllen auf den darunter befindlichen proximalen Klemmbügel 93 presst. Die über die Klemme 91 hinausstehenden freien Enden von Innen- und Außenhülle 3, 6 werden zusätzlich durch die Klammer 94 zusammengehalten und bspw. gegen unbeabsichtigtes Einreißen geschützt. Im Anschluss an die derartige Fixierung am proximalen Schaftende wird die Außenhülle 6 bis über das distale Schaftende abgerollt.

Weder der proximale Klemmbügel 93 noch der distale Klemmbügel 96 müssen zwischen zwei kontaminationsgeschützten Untersuchungen abgenommen werden. D.h. die Zeit, die zur Vorbereitung des Endoskops für eine weitere Untersuchung benötigt wird, ist vorteilhaft dadurch verkürzt, dass nach der Demontage des Kontaminationsschutzes die Klemmbügel samt darunter befindlicher Dichtungen auf dem Schaft verbleiben und zur Befestigung eines weiteren Kontaminationsschutzes verwendet werden können.

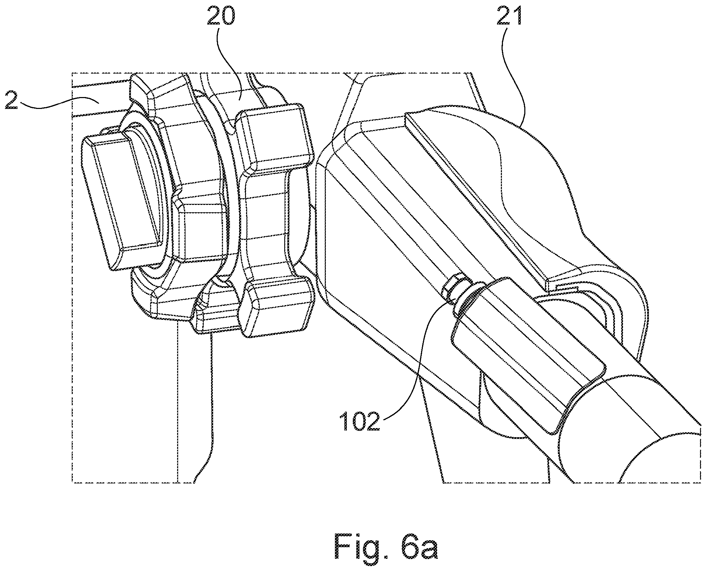

Die **Fig. 6a** bis **6c** zeigen Teilschritte der Anbringung einer Ausführungsform des erfindungsgemäßen Griffaufsatzes 21. Dieser ist typischerweise mindestens zweiteilig gefertigt, wobei das eine Teil ein in Fig. 6a abgebildetes ergonomisch geformtes Handstück 21 darstellt, welches seitlich an das Griffstück 2 des Endoskops angesetzt wird.

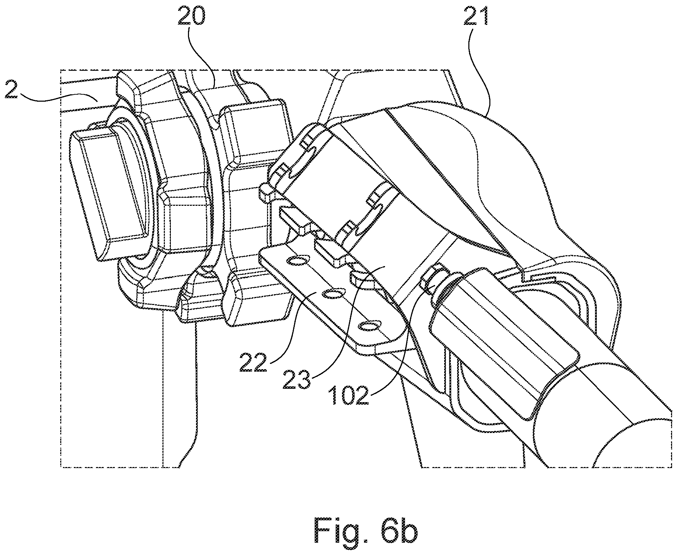

Diesem gegenüber wird in Fig. 6b ein Teil mit Ventilhalter 22 und Instrumentierhalter 23 als Gegenstück aufgesetzt und mit dem Griffstück 21 verbunden, etwa über eine Klickverbindung. Der Ventilhalter 22 ist gekennzeichnet durch Aufnahmen für drei Ventile, der Instrumentierhalter 23 bildet eine Aufnahme für den Zwei-Wege-Schlauchverteiler 24, der oberhalb der proximalen Instrumentierkanalöffnung 102 angeordnet wird.

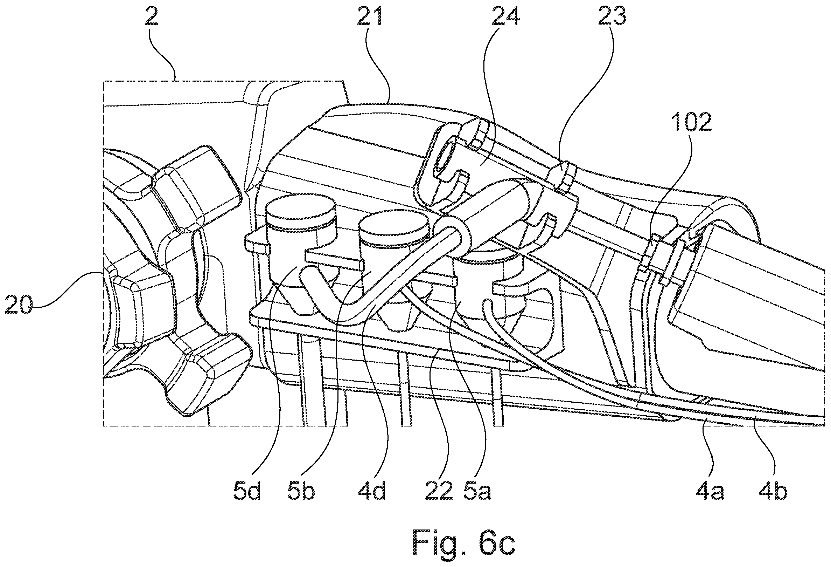

Fig. 6c zeigt den finalen Montagezustand. Die drei Ventile für Luft, Wasser und Absaugung 5a, 5b, 5c sind in die entsprechenden Aufnahmen am Ventilhalter 22 eingesetzt und mit den vom distalen Ende des Endoskopschafts 1 kommenden Luft- und Wasserschläuchen 4a, 4b sowie dem Saugschlauch 4d verbunden. Die aus den Ventilen auslaufenden Schläuche werden mit entsprechenden Vorrichtungen verbunden, mittels derer die Kanäle mit Überdruck, Unterdruck oder Wasser beaufschlagt werden können. Während der endoskopischen Untersuchung können die drei Ventile, 5a, 5b, 5d, durch den behandelnden Arzt per Fingerdruck geöffnet werden. In vorteilhafter Ausführungsform sind diese Ventile preisgünstige Einmalprodukte, die nach jeder Untersuchung entsorgt und gewechselt werden. Des Weiteren ist in Fig. 6c der y-förmige Zwei-Wege-Schlauchverteiler 24 abgebildet, der in den Instrumentierhalter 23 eingesteckt ist und mit dem aus der proximalen Instrumentierkanalöffnung 102 herausragenden Arbeitsschlauch 4c verbunden ist. An den seitlich auslaufenden Verteilerschenkeln ist der Saugschlauch 4d angeschlossen, der gradlinig verlaufende Schenkel des Verteilers dient als Zugang für im Zuge der Untersuchung zu verwendende Instrumente 80. Der Bereich zwischen dem distalen Ende des Zwei-Wege Schlauchverteilers 24 und der proximalen Instrumentierkanalöffnung 102, in welchem der Arbeitsschlauch 4c freiliegt, ist zum Durchtrennen des Arbeitsschlauch 4c im Zuge der Demontage nach erfolgter Endoskopie vorgesehen. Zum Durchtrennen sollte vorteilhaft eine Brennschere verwendet werden, welche die beiden durchtrennten Enden unmittelbar keimdicht verschmilzt, um ein Austreten der im Arbeitsschlauch 4c vorhandenen Verschmutzungen zu vermeiden.

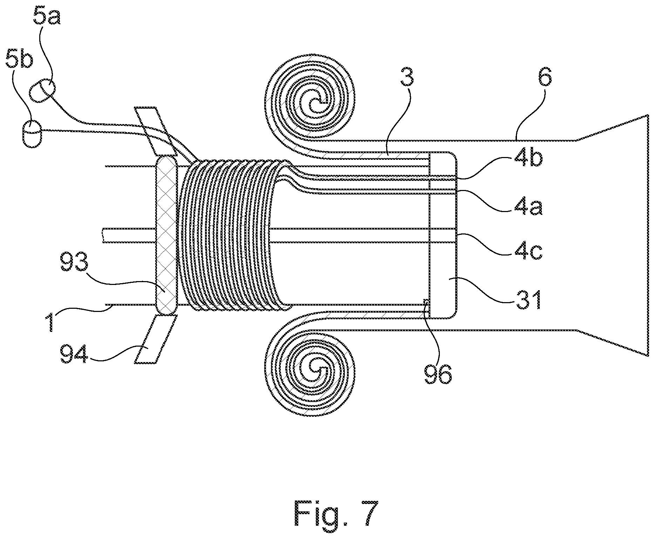

**Fig. 7** zeigt eine fertigungsseitig vormontierte Ausführungsform der Kontaminationsschutzvorrichtung zur raschen und bedienerfreundlichen Anbringung am Endoskopschaft 1. Dabei wird das Ziel verfolgt, die Vorrichtung in einem Stück auf den Schaft 1 aufzuziehen. Dazu liegen Innenhülle 3 und Außenhülle 6 aufeinander und sind gemeinsam aufgerollt bis an die Kappe 31. Dahinter sind die in der Stirnseite der Kappe 31 mündenden Luft- und Wasserschläuche 4a, 4b spiralförmig aufgewickelt, wobei die Wicklung einen größeren Durchmesser aufweist als der Schaft 1. Beide Schläuche 4a, 4b sind an der Klammer 94 fixiert und mit ihren proximalen Enden an Luftventil 5a und Wasserventil 5b angeschlossen. Zur Anbringung wird das proximale Ende das Arbeitsschlauchs 4c, welcher ebenfalls distal in der Stirnseite der Kappe 31 mündet, in die distale Instrumentierkanalöffnung 101 eingeführt und das gesamte vorgenannte Gebilde über die Schaftspitze auf den Schaft 1 geschoben, bis dass die Kappe 31 auf dem zuvor montierten distalen Klemmbügel 96 einrastet. Die Klammer 94 wird bis über den ebenfalls zuvor montierten proximalen Klemmbügel 93 hinaufgeschoben und dabei Innenhülle 3 und Außenhülle 6 abgerollt. Es müssen anschließend nur noch die proximalen Enden der beiden Hüllen 3, 6 in die Klammer 94 eingeschoben werden und die Klemme 91 zur Fixierung und Abdichtung im Bereich des proximalen Klemmbügels 93 darum geschlossen werden. Zuletzt werden Luftventil 5a und Wasserventil 5b am Griffaufsatz 21 befestigt und der aus der proximalen Instrumentierkanalöffnung 102 herausragende Arbeitsschlauch an den Zwei-Wege-Schlauchverteiler 24 angeschlossen werden.

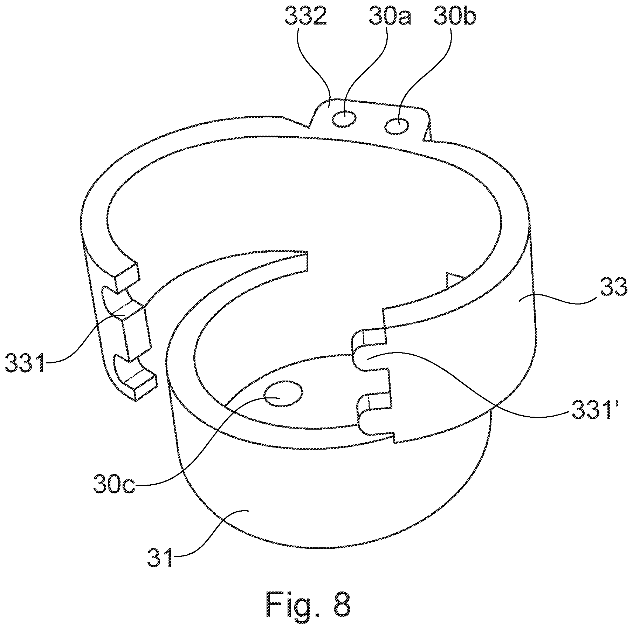

**Fig. 8** zeigt eine vorteilhafte Ausführungsform der Kappe 31, welche zur Verwendung ohne distalen Klemmbügel geeignet ist. Dazu ist an das proximale Ende der Kappe 31 ein bügelartiger Klemmer 33 angeformt. Dieser ist aus einem geeignet verformbaren Kunststoff gefertigt und kann um den Endoskopschaft zusammengebogen und über den Klickverschluss 331, 331' fixiert werden. Luftschlauchöffnung 30a und Wasserschlauchöffnung 30b sind hier röhrenförmig in einem Steg 332 angeordnet, welcher Klammer 31 und Klemmer 33 verbindet. Der Vorteil dieser Ausführungsform besteht in ihrem geringeren Durchmesser im Vergleich zu der Variante mit distalem Klemmbügel. Die in Fig. 8 dargestellte Kappe 31 mit Klemmer 33 kann vorteilhaft auch in einer fertigungsseitig vormontierten Ausführungsform der Kontaminationsschutzvorrichtung entsprechend Fig. 7 Verwendung finden. Zudem kann das distale Ende der Kappe auch aus einem optisch neutralen Schrumpfschlauch gefertigt sein.

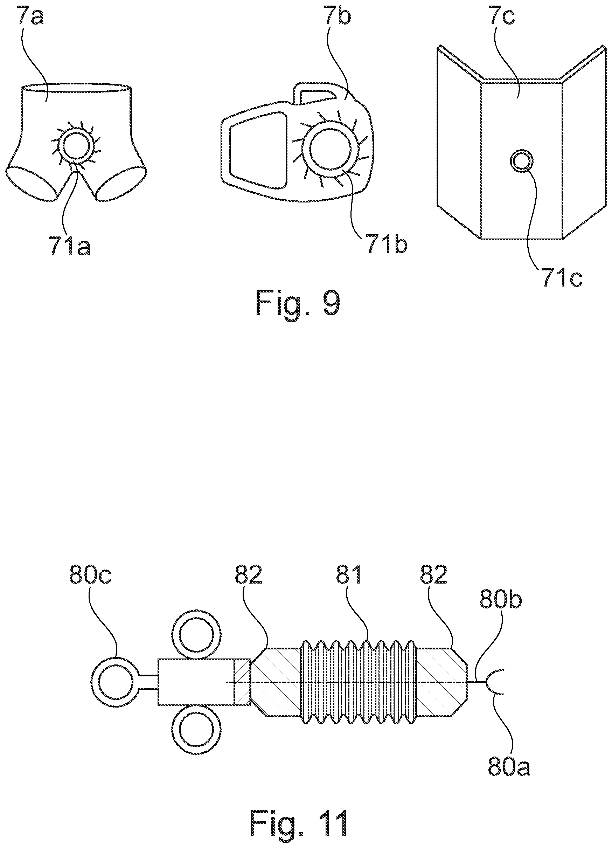

In **Fig. 9** sind die in den Ansprüchen 2 bis 4 beschriebenen Gegenstände abgebildet, die dem patientenseitigen Anschluss des distalen Endes der Außenhülle 6 dienen. Dies ist zum einen die erfindungsgemäße Windelhose 7a, welche bei Koloskopien Verwendung findet. Diese Windelhose 7a wird von dem zu untersuchenden Patienten angelegt und weist eine Öffnung mit einem Anschlussteil 71a auf, welche geeignet zu der zu untersuchenden Körperöffnung, d. h. Anus oder Enterostoma, positioniert ist. An dieses Anschlussteil 71a kann die Außenhülle 6 mittels des in vorteilhafter Ausführungsform an ihrem distalen Ende vorgehaltenen Anschlussstücks 61a angeschlossen werden, so dass eine luft-, wasser- und keimdichte Verbindung entsteht. Im Zuge der Koloskopie wird dann der von der Innenhülle 3 geschützte Endoskopschaft 1 durch die an der Windelhose 7a vorgehaltene Öffnung durchgeschoben und in die zu untersuchende Körperöffnung des Patienten eingeführt. Der während der Untersuchung austretende Darminhalt bzw. sonstige Körperflüssigkeiten werden somit durch die Windelhose 7a einerseits und das zwischen Außen- und Innenhülle 6, 3 gebildete Schutzvolumen andererseits von einem Austreten in die Untersuchungsumgebung abgehalten.

Für Gastroskopien findet der erfindungsgemäße Beißring 7b Verwendung, an dessen Öffnung das Anschlussteil 71b vorgehalten ist. Dieses dient wiederrum dem patientenseitigen Anschluss der Außenhülle 6 über das Anschlussstück 61b. Um während der Untersuchung das Ausrinnen von Speichel aus den Mundwinkeln des Patienten und damit einhergehende Kontamination der Außenseite der Außenhülle 6 zu vermeiden oder minimieren, kann der Beißring 7b noch um Abschnitte erweitert werden, die das Gesicht oder zumindest die Kieferpartie des Patienten maskenartig umschließen.

Alternativ zu Windelhose 7a oder Beißring 7b kann eine Trennwand 7c mit Öffnung und Anschlussteil 71c verwendet werden, hinter der der Patient während der endoskopischen Untersuchung positioniert wird. Der behandelnde Arzt agiert von der Vorderseite der Trennwand und ist somit von unmittelbarer Kontamination geschützt. In weiterer vorteilhafter Ausführungsform kann die Trennwand 7c die Form einer bis auf die Durchführungsöffnung geschlossenen Kabine annehmen, in welcher dann sämtliche während der Untersuchung aus dem Patienten geförderten Kontaminationen eingeschlossen bleiben.

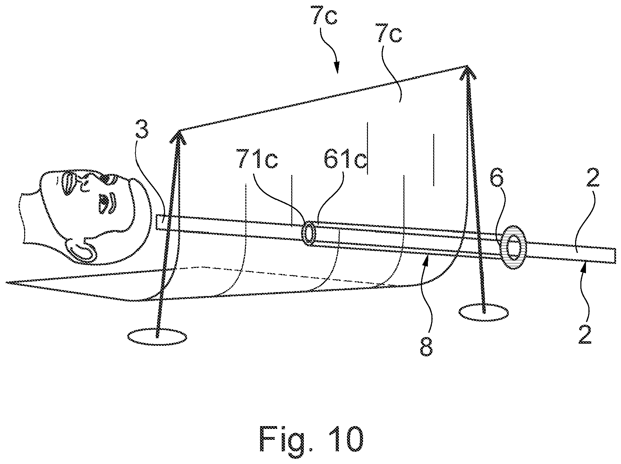

**Fig. 10** zeigt eine Ausführungsform des erfindungsgemäßen Endoskops, bei der eine aufgespannte Folie 7c als Abtrennung zwischen Patient und behandelndem Arzt verwendet wird. Diese Folie ist mit einer Öffnung versehen an welche die Außenhülle 6 mit ihrem distalen Ende angeschweißt ist, wodurch hier eine luft-, wasser- und keimdichte Verbindung realisiert wird. Die trennende Folie 7c sollte derart dimensioniert sein, dass sie umgeschlagen und unter den zu untersuchenden Patienten gelegt werden kann. Dadurch werden alle während der Untersuchung auftretenden Verschmutzungen auf der Folie gesammelt und vom Rest der Untersuchungsumgebung, insbesondere der Patientenliege, ferngehalten.

**Fig. 11** zeigt den erfindungsgemäßen Instrumentenschutz 8 nebst einem durchgeführten Instrument 80, welches üblicherweise aus einem Mikrogerät 80a an einem Instrumentenbowdenzug 80b besteht und mit dem Bedienelement 80c bedient werden kann. Der Instrumentenschutz 8 besteht aus einem hier balgartig aufgeschobenen Instrumentenschlauch 81, der an beiden Enden von den Instrumentenschlauchkappen 82 begrenzt ist, an deren Stirnseiten jeweils Öffnungen zur Durchführung des Instruments 80 vorgehalten sind. Während der endoskopischen Untersuchung ist der Instrumentenschutz 8 im dargestellten balgartig zusammengerafften Zustand an das proximale Ende des gradlinigen Schenkels des Zwei-Wege Schlauchverteilers 24 angeschlossen. Um nach erfolgtem chirurgischen Eingriff die verschmutzten Teile des Instruments 80 aus dem Arbeitsschlauch 4c zu entnehmen ohne dabei Kontaminationen freizusetzen, wird es in den Instrumentenschlauch 81 hineingezogen, welcher zu diesem Zwecke zu voller Länge ausgezogen wird. Die Länge des Instrumentenschlauchs 81 ist dementsprechend so zu dimensionieren, dass das Mikrogerät 80a nebst Bowdenzug 80b vollständig von ihm umhüllt werden kann. Nach Einziehen des Instruments 80 in den Instrumentenschutz 8 kann dieser vom Zwei-Wege Schlauchverteiler 24 entfernt werden, wobei nun alle Verschmutzung im Inneren des Instrumentenschlauchs 81 zurückgehalten werden.

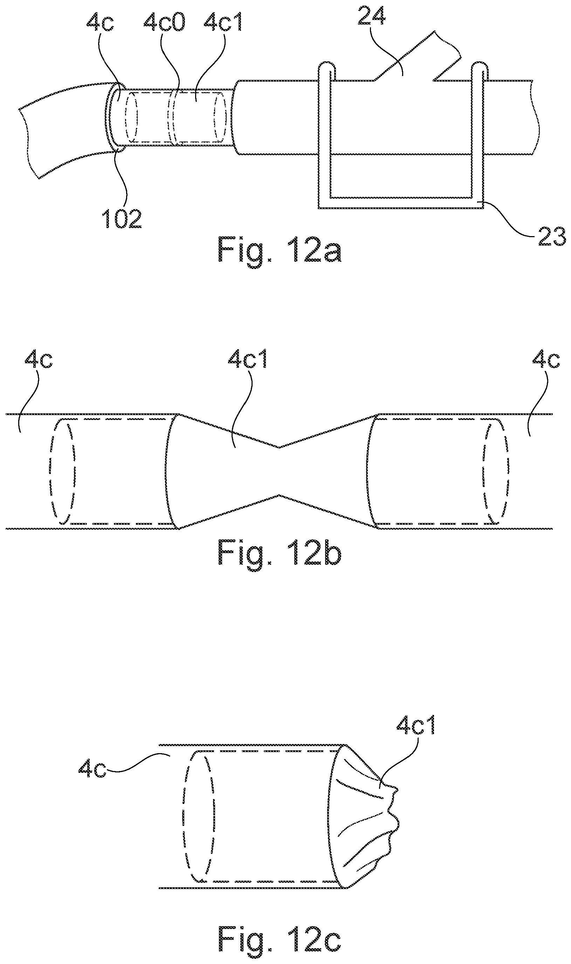

**Fig. 12a** bis **12c** zeigen eine vorteilhafte Ausführungsform des erfindungsgemäßen Arbeitsschlauchs 4c mit einem Schutzschlauch 4c1, welcher einer kontaminationsgeschützten Demontage zuträglich ist.

Fig. 12a zeigt den montierten Arbeitsschlauch 4c, der aus der proximalen Instrumentierkanalöffnung 102 herausragt und an das distale Ende des Zwei-Wege-Schlauchverteilers 24 im Instrumentierhalter 23 angeschlossen ist. In diesem frei zugänglichen Abschnitt weist der Arbeitsschlauch 4c eine Sollbruchstelle 4c0, etwa in Form einer umlaufenden Kerbung, auf. Vor und hinter dieser Sollbruchstelle 4c0 ist der Schutzschlauch 4c1 auf dem gesamten Umfang luft-, wasser- und keimdicht mit Außenseite des Arbeitsschlauchs 4c verbunden, typischerweise durch eine Schweiß- oder Klebeverbindung..

In Fig. 12b ist dargestellt, wie der Schutzschlauch nach Aufbrechen der Sollbruchstelle 4c0 durch Ziehen an den beiden getrennten Enden des Arbeitsschlauchs 4c in die Länge gezogen wird, wodurch er schließlich zerrissen wird.

Der für den Schutzschlauch 4c1 verwendete polymere Werkstoff sollte eine wesentlich höhere Reißdehnung aufweisen als der Werkstoff des Arbeitsschlauchs 4c, so dass die auseinander gerissenen Endabschnitte wie in Fig. 12c dargestellt so stark plastisch verformt sind, dass sie die Enden des Arbeitsschlauchs 4c taschenartig verschließen und vor Austreten von Kontaminationen schützen.

Anstelle der hier dargestellten Ausführungsform mit an der Außenseite des Arbeitsschlauchs 4c befestigtem Schutzschlauch 4c1, könnte dieser auch sinngemäß im Inneren des Arbeitsschlauchs angebracht sein.

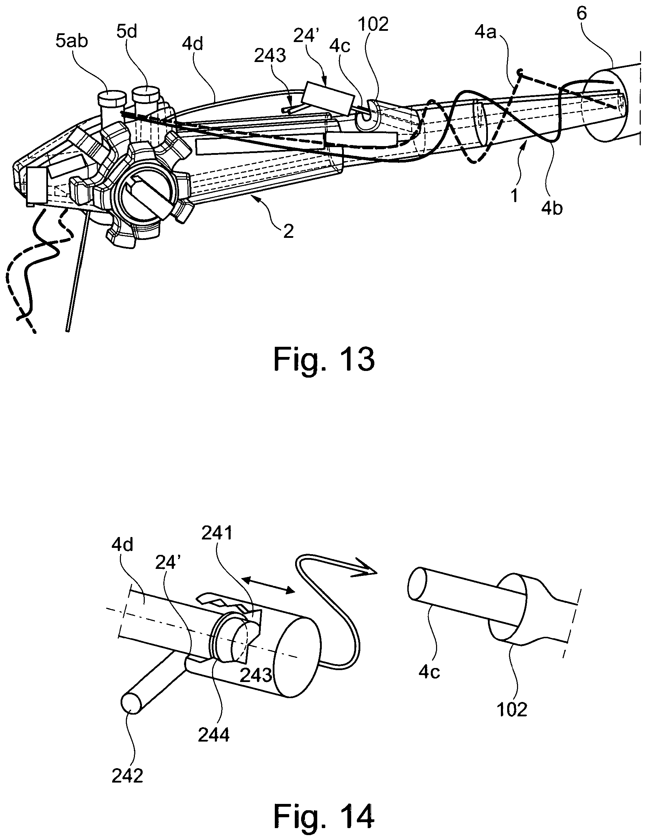

**Figur 13** zeigt eine perspektivische Gesamtansicht einer weiteren Ausführungsform des erfindungsgemäßen Endoskops mit Kontaminationsschutzvorrichtung, bei welcher der Zwei-Wege-Schlauchverteiler direkt an die proximale Instrumentierkanalöffnung angeschlossen ist.

Anstatt einen Griffaufsatz zur Befestigung des Zwei-Wege-Schlauchverteilers und der Ventile einzusetzen, ist der Schlauchverteiler 24' direkt an die proximale Instumentierkanalöffnung 102 angeschlossen. Die Luft- und Wasserventile sind in einem einzigen Ventil 5ab kombiniert, welches zusammen mit dem Absaugventil 5d in hierfür im Griffstück 2 vorhandene Halterungen eingesetzt sind.

An den Zwei-Wege-Schlauchverteiler 24' sind einmal das aus der proximalen Instrumentierkanalöffnung 102 herausgeführte proximale Ende des Arbeitsschlauches 4c und weiterhin der zum Absaugventil 5d führende Absaugschlauch 4d angeschlossen. Die Schläuche für die Druckluft 4a und die Wasserversorgung 4b sind zur Sicherung um den Schaft 1 gewickelt und zum kombinierten Luft-/Wasserventile 5ab geführt und dort angeschlossen.

In **Figur 14** ist der zum direkten Anschluss an die Instrumentierkanalöffnung geeignete Zwei-Wege-Schlauchverteiler der Ausführungsform aus Figur 13 schematisch im Detail dargestellt.

Der y-förmige Zwei-Wege-Schlauchverteiler 24' verfügt über drei Enden bzw. Anschlüsse. Mit dem distalen Ende 241 ist, welches insbesondere konisch geformt sein kann, ist er in das proximale Ende 242 des aus der Instrumentierkanalöffnung 102 herausgeführte Arbeitsschlauchs 4c einsteckbar. An einem der beiden proximalen Enden ist der Absaugschlauch 4d angeschlossen, das andere Ende 242 ist offen und dient als Instrumentenzugang.

Zur Befestigung an der proximalen Instrumentierkanalöffnung ist der Schlauchverteiler 24' mit einer Ringmutter 243 ausgestattet, welche axial verschiebbar auf dem Schlauchverteiler 24' sitzt. Ein im Bereich des distalen Endes 241 angeformter und radial hervorstehender Kragen 244 wirkt mit einem Anschlag der Ringmutter 243 zusammen um den Schlauchverteiler nach der Fixierung der Ringmutter an einem entsprechenden Anschlussmittel der proximalen Instrumentierkanalöffnung 102 auf diesem zu befestigen. Die Instrumentierkanalöffnung 102 kann hierzu beispielsweise einen weit verbreiteten Luerlock-Anschluss aufweisen.

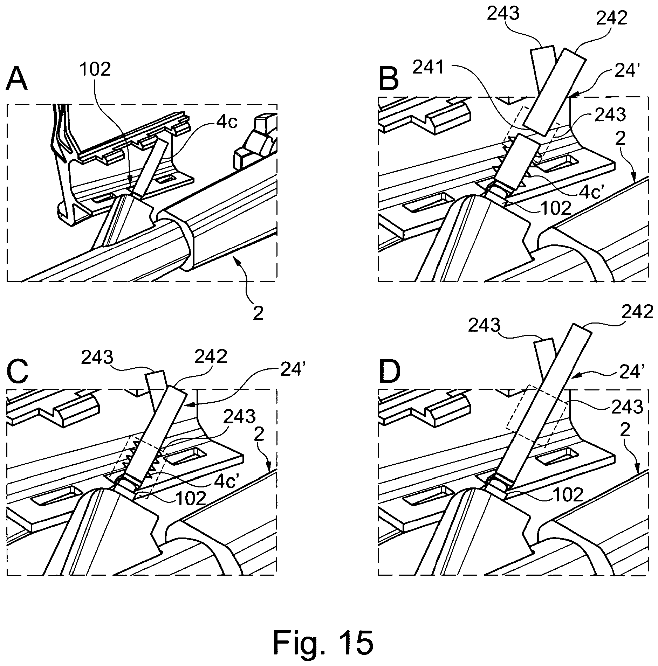

Die (Teil-)**Figuren 15A** - **D** illustrieren das Vorgehen beim direkten Anschließen eines hierfür geeigneten Schlauchverteilers der Ausführungsform gemäß der Figuren 13 und 14.

Zunächst wird wie in Teilfigur A gezeigt der Arbeitschlauch 4c ausreichend weit aus der proximalen Instrumentierkanalöffnung 102 herausgeschoben. Dann wird der Zwei-Wege-Schlauchverteiler 24' mit seinem distalen Ende 41 durch Einstecken mit dem Arbeitsschlauch verbunden, Teil-figur B. Der Arbeitsschlauch 4c ist hier so ausgebildet, dass durch die beim Einstecken des Zwei-Wege-Schlauchverteilers 24' ausgeübten Druckkräfte das proximale Arbeitsschlauchende faltenbalgartig zusammengestaucht und so der Faltenbalganschnitt 4c' gebildet wird. Teilfigur C zeigt den Schritt in dem die Ringmutter 243 des Schlauchverteilers 24' mithilfe eines Anschlussmittels, hier eines Luerlock-Anschlusses, an der proximalen Instrumentierkanalöffnung angeschlossen wird. Nun ist das Endoskop bereit für eine Untersuchung oder Operation. Nach deren Ende wird wie in Teilfigur D angedeutet der Zwei-Wege-Schlauchverteiler 24' wieder gelöst und mitsamt dem Arbeitskanal von der proximalen Instrumentierkanalöffnungen 102 weg gezogen, wodurch sich der Faltenbalgabschnitt 4c' des Arbeitskanals 4c wieder entfaltet. Mit einer Heißschere kann nun der Arbeitsschlauch in einem Schritt durchtrennt und versiegelt und so kontaminationsfrei nach distal aus dem Instrumentierkanal herausgezogen werden.

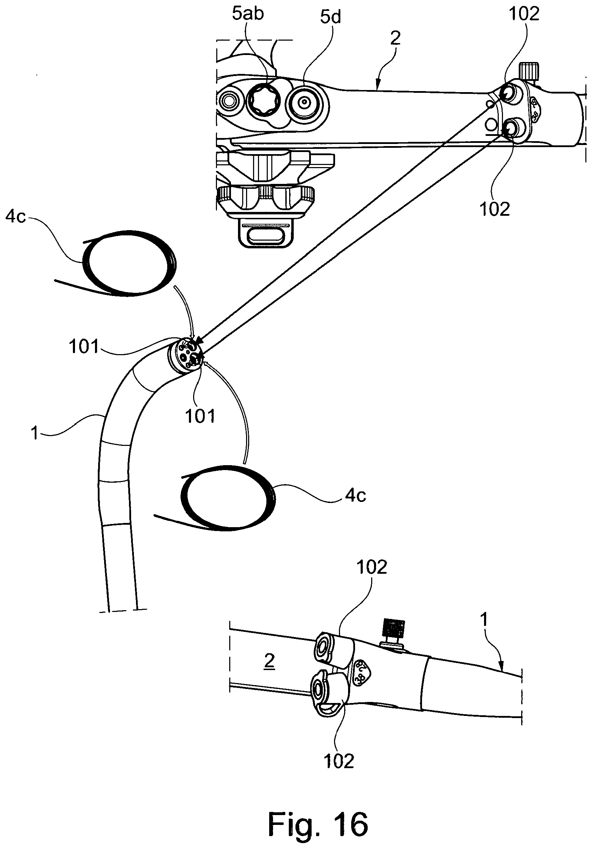

**Figur 16** zeigt eine weitere Ausführungsform eines erfindungsgemäßen Endoskops mit zwei Instrumentierkanälen und entsprechend zwei Arbeitsschläuchen.

Das in der linken Teilfigur gezeigte Distale Ende des Schaftes 1 verfügt über zwei distale Instrumentierkanalöffnungen 101, in welche im Rahmen des erfindungsgemäßen Kontaminationsschutzes je ein Arbeitsschlauch 4c zu schieben ist. Die zwei Instrumentierkanäle des her gezeigten Endoskops münden proximal in zwei entsprechenden Instrumentierkanalöffnungen 102, Teilfigur rechts oben. In dieser Teilfigur sind auch die Ventilhalterungen mit Ventilen 5ab für Luft- und Wasser sowie 5d für die Absaugung dargestellt. In der Teilfigur rechts unten sind die Instrumentierkanalöffnungen 102 mit Schutzkappen verschlossen dargestellt.

### Bezugszeichenliste

- 1: Schaft
- 10: Instrumentierkanal
- 101: Instrumentierkanalöffnung distal
- 102: Instrumentierkanalöffnung proximal
- 11: Optik
- 12: Beleuchtungsvorrichtung
- 13: Düse
- 2: Griffstück
- 20: Bedienelement Schaftspitzenabwinklung
- 21: Griffaufsatz
- 22: Ventilhalter
- 23: Instrumentierhalter
- 24, 24': 2-Wege-Schlauchverteiler
- 241: distales Ende von 24 und 24'
- 242: Instrumentenöffnung von 24'
- 243: Ringmutter
- 3: Innenhülle
- 30: Innenhülle Stirnseite
- 30a: Luftschlauchöffnung
- 30b: Wasserschlauchöffnung
- 30c: Arbeitsschlauchöffnung
- 31: Kappe
- 32: Gewebeschlauch
- 33: Klemmer
- 331, 331': Klickverschluss
- 332: Steg
- 4a: Luftschlauch
- 4b: Wasserschlauch
- 4c: Arbeitsschlauch
- 4c': Faltenbalgabschnitt
- 4c0: Sollbruchstelle
- 4c1: Schutzschlauch
- 4d: Saugschlauch
- 5a: Luftventil
- 5b: Wasserventil
- 5ab: kombiniertes Luft- und Wasserventil
- 5d: Saugventil
- 6: Außenhülle
- 61a,b,c: Anschlussstücke
- 7a: Windelhose
- 7b: Beißring
- 7c: Trennwand
- 71a,b,c: Anschlussteile
- 8: Instrumentenschutz
- 81: Instrumentenschlauch
- 82: Instrumentenschlauchkappe
- 80: Instrument
- 80a: Mikrogerät
- 80b: Instrumentenbowdenzug
- 80c: Bedienelement Instrument
- 91: Klemme
- 92: Dichtung proximal
- 93: Klemmbügel proximal
- 94: Klammer
- 95: Dichtung distal
- 96: Klemmbügel distal
- 960: Positionierhilfe

## Patentansprüche

1. Endoskop mit Kontaminationsschutzvorrichtung umfassend
- einen starren oder flexiblen Schaft (1), an dessen Spitze eine Optik (11), eine Beleuchtungsvorrichtung (12) und eine Düse (13) angeordnet sind,
- ein an den Schaft (1) anschließendes Griffstück (2),
- eine Innenhülle (3), die an ihrem einen distalen Ende geschlossen und zumindest stirnseitig optisch transparent ist und die entlang des Schafts (1) kondomartig abgerollt ist,
- jeweils mindestens einen Luftschlauch (4a) und einen Wasserschlauch (4b), die am distalen Ende der Innenhülle (3) offen enden und mit diesem verbunden sind und die zwischen Schaft (1) und Innenhülle (3) positioniert sind, und
wobei eine Außenhülle (6) die Innenhülle (3) umhüllt und an einem proximalen Ende der Innenhülle (3) mit selbiger luft-, wasser- und keimdicht verbunden ist.

2. Endoskop nach Anspruch 1, **dadurch gekennzeichnet, dass** an einem distalen Ende der Außenhülle (6)
- ein erstes Anschlussstück (61a) angeformt ist, und eine Windelhose (7a) für einen koloskopisch zu untersuchenden Patienten vorgehalten wird, welche eine Öffnung mit einem ersten Aufnahmeteil (71a) aufweist, an welches das erste Anschlussstück (61a) der Außenhülle (6) luft-, wasser- und keimdicht angeschlossen ist, und/oder
- ein zweites Anschlussstück (61b) angeformt ist, und ein Beißring (7b) für einen gastroskopisch zu untersuchenden Patienten vorgehalten wird, welcher eine Öffnung mit einem zweiten Aufnahmeteil (71b) aufweist, an welches das zweite Anschlussstück (61b) der Außenhülle (6) luft-, wasser- und keimdicht angeschlossen ist, und/oder
- ein drittes Anschlussstück (61c) angeformt ist, und eine Trennwand (7c) oder Kabine für einen dahinter bzw. darin zu positionierenden, zu untersuchenden Patienten vorgehalten wird, welche eine Öffnung mit einem dritten Aufnahmeteil (71c) aufweist, an welches das dritte Anschlussstück der Außenhülle (6) luft-, wasser- und keimdicht angeschlossen ist.

3. Endoskop nach Anspruch 1, **dadurch gekennzeichnet, dass** eine Folie mit einer Öffnung an ein distales Ende der Außenhülle (6) luft-, wasser- und keimdicht angeschweißt und mittels einer Haltevorrichtung vor einem zu untersuchenden Patienten aufgespannt ist.

4. Endoskop nach einem der vorhergehenden Ansprüche, **dadurch gekennzeichnet, dass** Innenhülle (3) und Außenhülle (6)
- aus luft- und wasserdichtem sowie für Krankheitskeime undurchlässigem Material bestehen, und/oder
- aus flexibel komprimierbarem Material bestehen, d.h. insbesondere aufrollbar oder balgartig faltbar sind.

5. Endoskop nach einem der Ansprüche 1 - 4, **dadurch gekennzeichnet, dass**
- im Schaft (1) mindestens ein Instrumentierkanal (10) verläuft, welcher an der Schaftspitze in jeweils einer distalen Instrumentierkanalöffnung (101) und am Griffstück (2) in einer proximalen Instrumentierkanalöffnung (102) mündet,
- die Innenhülle (3) stirnseitig eine zur distalen Instrumentierkanalöffnung (101) kongruente Arbeitsschlauchöffnung (30c) aufweist, und
- an die Arbeitsschlauchöffnung (30c) keimdicht anschließend ein Arbeitsschlauch (4c) angeformt ist, welcher im Instrumentierkanal (10) positioniert ist und aus der proximalen Instrumentierkanalöffnung (102) herausragt.

6. Endoskop nach Anspruch 5, **dadurch gekennzeichnet, dass** der Arbeitsschlauch (4c) in seinem aus der proximalen Instrumentierkanalöffnung (102) herausragenden Abschnitt eine Sollbruchstelle (4c0) aufweist, und dass ein Schutzschlauch (4c1) aus einem polymeren Werkstoff, welcher eine höhere Reißdehnung als ein Werkstoff des Arbeitsschlauchs (4c) aufweist, derart an einer Außen- oder Innenseite des Arbeitsschlauchs (4c) befestigt ist, dass der Schutzschlauch (4c1) sowohl vor als auch hinter der Sollbruchstelle (4c0) luft-, wasser- und keimdicht mit dem Arbeitsschlauch (4c) verbunden ist.

7. Endoskop nach einem der Ansprüche 1 - 6, **gekennzeichnet durch**
- Ventilhalterungen (22a, 22b) im Griffstück (2), in welche ein Luftventil (5a) und ein Wasserventil (5b) oder ein in einem Ventilblock (5ab) integriertes kombiniertes Luft-/Wasserventil (5ab) sowie ein Absaugventil (5d) einsteckbar sind, und
- einen Zwei-Wege-Schlauchverteiler (24') der mit einem distalen, insbesondere konisch geformten, Ende (241) in ein proximales Ende des Arbeitsschlauchs (4c) eingesteckt ist, wobei der Schlauchverteiler (24') weiterhin insbesondere eine Instrumentieröffnung (242) und eine axial relativ zum Schlauch (4c) verschiebbare Ringmutter (243) aufweist, mittels welcher an einer proximalen Instrumentierkanalöffnung (102) des Endoskops befestigbar ist.

8. Endoskop nach einem der Ansprüche 1 - 6, **gekennzeichnet durch** einen auf das Griffstück (2) aufgesteckten Griffaufsatz (21), in welchem vorgehalten sind:
- ein Luftventil (5a) und ein Wasserventil (5b) in einem Ventilhalter (22),
- ein Instrumentierhalter (23) mit einem darin eingelegten 2-Wege-Schlauchverteiler (24), an dessen einem distalen Ende der Arbeitsschlauch (4c) angeschlossen ist,
- ein Saugventil (5d), und
- ein Saugschlauch (4d), der an einem ersten proximalen Ende des Schlauchverteilers (24) und dem Saugventil (5d) angeschlossen ist

9. Endoskop nach Anspruch 8, **dadurch gekennzeichnet, dass** an einem zweiten proximalen Ende des Schlauchverteilers (24) ein Instrumentenschutz (8) angeschlossen ist, welcher aus einem balgartig raffbaren Instrumentenschlauch (81) besteht, der an jedem Ende eine Instrumentenschutzkappe (82) mit stirnseitiger Öffnung aufweist, und eine Länge aufweist, die größer ist als eine Länge des Arbeitsschlauchs (4c).

10. Endoskop nach einem der vorhergehenden Ansprüche, **dadurch gekennzeichnet, dass** die Innenhülle (3) und/oder die Außenhülle (6) an einem proximalen Ende des Schafts (1) an einer Mantelfläche des Schafts (1) befestigt ist, insbesondere dadurch dass sie durch eine Klemme (91) oder einen Gummiring auf einen auf der Mantelfläche des Schafts (1) angeordneten und mit einer proximalen Dichtung (92) unterlegten, proximalen Klemmbügel (93) gepresst werden, welcher Durchführungen für Luftschlauch (4a) und Wasserschlauch (4b) aufweist.

11. Endoskop nach einem der vorhergehenden Ansprüche, **dadurch gekennzeichnet, dass** das distale Ende der Innenhülle (3) als eine im Wesentlichen hohlzylindrische Kappe (31) mit einer offenen Stirnseite ausgebildet ist, die an einer der offenen Stirnseite gegenüberliegenden Stirnseite eine Luftschlauchöffnung (30a) und eine Wasserschlauchöffnung (30b) aufweist, an die Luftschlauch (4a) und Wasserschlauch (4b) angeformt sind, wobei
- an die Kappe (31) ein bügelartiger Klemmer (33) mit einem Klickverschluss (331, 331') angeformt ist und die Befestigung der Kappe (31) auf dem distalen Ende des Schafts (1) dadurch gewährleistet ist, dass sie auf einem auf einer Mantelfläche des Schafts (1) angeordneten und mit einer distalen Dichtung (95) unterlegten, distalen Klemmbügel (96) aufsitzt, welcher Durchführungen für Luftschlauch (4a) und Wasserschlauch (4b) aufweist, oder
- an eine offene Stirnseite der Kappe (31) zur Klemmung auf dem distalen Ende des Schaftes axial verlaufende elastische Branchen angeformt sind.

12. Endoskop nach einem der vorhergehenden Ansprüche, **dadurch gekennzeichnet, dass** Innenhülle (3) und Außenhülle (6) einteilig gefertigt sind, d.h. dass die luft-, wasser- und keimdichte Verbindung der Außenhülle (6) mit dem proximalen Ende der Innenhülle (3) bereits fertigungsseitig vorgehalten wird, bspw. durch Verschweißen und/oder die Innenhülle (3) mit einem Gewebeschlauch unterlegt ist und/oder die Außenhülle (6) das Griffstück (2) und den Griffaufsatz (21) umhüllt.

13. Endoskop nach den Ansprüchen 8 und 12, **dadurch gekennzeichnet, dass** der Zwei-Wege-Schlauchverteiler (24) in den das Griffstück (2) und den Griffaufsatz (21) umhüllenden Abschnitt der Außenhülle (6) integriert ist.

14. Verfahren zur Anbringung einer Kontaminationsschutzvorrichtung für ein Endoskop nach Anspruch 1, **gekennzeichnet durch** die Schritte:
- Beaufschlagen des optisch transparenten Bereichs am distalen Ende der Innenhülle (3) mit einem Stoff, der optischen Kontakt mit der Optik (11) des Endoskops herstellt,
- Aufstecken des Griffaufsatzes (21) auf das Griffstück (2),
- Einführen des distalen Endes des Endoskopschafts (1) in die aufgerollte Innenhülle (3),
- kondomartiges Abrollen der Innenhülle (3) unter Einschluss des Schafts (1) und des daran positionierten Luftschlauchs (4a) und Wasserschlauchs (4b),
- Verbinden der proximalen Enden von Luftschlauch (4a) und Wasserschlauch (4b) mit Luftventil (5a) und Wasserventil (5b) am Griffaufsatz (21),
- Aufschieben der aufgerollten Außenhülle (6) über die Innenhülle (3) und luft-, wasser- und keimdichte Befestigung am proximalen Ende der Innenhülle (3), und
- kondomartiges Abrollen der Außenhülle (6) bis über die Spitze des Endoskopschafts (1) hinaus

15. Verfahren zur Anbringung einer Kontaminationsschutzvorrichtung für ein Endoskop nach Anspruch 2 gemäß der Verfahrensschritte nach Anspruch 14, **dadurch gekennzeichnet, dass** anschließend das erste oder zweite oder dritte Anschlussstück (61a,b,c) der Außenhülle (6) mit dem ersten oder zweiten oder dritten Anschlussteil (71a,b,c) verbunden wird.

16. Verfahren zur Anbringung einer Kontaminationsschutzvorrichtung für ein Endoskop nach Anspruch 11 gemäß der Verfahrensschritte nach einem der Ansprüche 14 oder 15, **dadurch gekennzeichnet, dass** zur Befestigung und Verbindung von Innenhülle (3) und Außenhülle (6) folgende Schritte durchgeführt werden:
- Aufkleben der proximalen Dichtung (92) auf die Mantelfläche des proximalen Endes des Schafts (1),
- Anbringen des proximalen Klemmbügels (93) auf der proximalen Dichtung (92),
- Aufkleben der distalen Dichtung (95) auf die Mantelfläche des distalen Endes des Schafts (1),
- Anbringen des distalen Klemmbügels (96) auf der distalen Dichtung (95) unter Verwendung einer Positionierhilfe (960),
- Positionieren von Luftschlauch (4a) und Wasserschlauch (4b) in den Durchführungen am distalen Klemmbügel (96),
- Aufstecken der Kappe (31) auf den distalen Klemmbügel (96),
- Positionieren von Luftschlauch (4a) und Wasserschlauch (4b) in den Durchführungen am proximalen Klemmbügel (93),
- Positionieren der proximalen Enden von Innenhülle (3) und Außenhülle (6) über dem proximalen Klemmbügel (93), und
- Einklemmen von Innenhülle (3) und Außenhülle (6) zwischen dem proximalen Klemmbügel (93) und der Klemme (91) oder dem Gummiring

17. Verfahren zur Anbringung einer Kontaminationsschutzvorrichtung für ein Endoskop nach Anspruch 11 gemäß der Verfahrensschritte nach einem der Ansprüche 14 - 16, **dadurch gekennzeichnet, dass** vor dem Einführen des distalen Endes des Schafts (1) in die aufgerollte Innenhülle (3) das proximale Ende des Arbeitsschlauchs (4c) soweit in die distale Instrumentierkanalöffnung (101) eingeführt wird, dass es aus der proximalen Instrumentieröffnung (102) hervorragt, und dass anschließend
- das proximale Ende des Arbeitsschlauchs (4c) mit dem distalen Ende eines 2-Wege-Schlauchverteilers (24, 24'),
- das erste proximale Ende des Schlauchverteilers (24, 24') mit dem Saugventil (5d) mittels des Saugschlauchs (4d), und
- das zweite proximale Ende des Schlauchverteilers (24, 24') mit dem balgartig gerafften Instrumentenschutz (8)
verbunden wird.

18. Verfahren nach Anspruch 17 **dadurch gekennzeichnet, dass** der Schlauchverteiler (24') mittels einer Ringmutter (243) direkt an der proximalen Instrumentierkanalöffnung (102) befestigt wird.

19. Verfahren zum Vorbereiten eines Endoskop gemäß einem der Ansprüche 1 - 13 zu einer zweiten kontaminationsgeschützten endoskopischen Untersuchung nach der kontaminationsgeschützten Durchführung einer ersten endoskopischen Untersuchung deren letzter Schritt in dem Herausziehen des Endoskopschafts aus der untersuchten Körperöffnung besteht,
**gekennzeichnet durch** die Schritte:
- Durchtrennen der Außenhülle (6) im Bereich ihres distalen Endes sowie von Luftschlauch (4a) und Wasserschlauch (4b) im Bereich des Griffstücks (2) jeweils mit einer Brennschere, und
- Demontage und Entsorgung der Kontaminationsschutzvorrichtung, wobei gegebenenfalls auf der Mantelfläche des Schaftes befestigte Klemmbügel (93, 96) am Endoskop belassen werden,
- Bereitstellung eines weiteren Kontaminationsschutzes umfassend Innenhülle (3), Außenhülle (6) sowie Wasser-, Luft- und Arbeitschläuche (4a, 4b, 4c), und
- Montage des weiteren Kontaminationsschutzes gemäß einem der Ansprüche 14 - 18, wobei gegebenenfalls das Anbringen des proximalen Klemmbügels (93) und des distalen Klemmbügels (96) als redundant entfällt.

## Claims

1. Endoscope with contamination protection device comprising
- a rigid or flexible shaft (1), at the tip of which an optic (11), a lighting device (12) and a nozzle (13) are arranged,
- a handle (2) attached to the shaft (1),
- an inner sheath (3) which is closed at its one distal end and is optically transparent at least on the front side and which is condom-like rolled out along the shaft (1),
- in each case at least one air hose (4a) and one water hose (4b), which end openly at the distal end of the inner sheath (3) and are connected to the latter and which are positioned between the shaft (1) and the inner sheath (3), and
wherein an outer sheath (6) envelops the inner sheath (3) and is connected to it at a proximal end of the inner sheath (3) in an air, water and germ-tight manner.

2. Endoscope according to Claim 1, **characterized in that** at a distal end of the outer sheath (6)
- a first connection piece (61a) is integrally formed, and a pair of diapers (7a) for a patient to be examined colonoscopically is provided, which has an opening with a first receiving part (71a) to which the first connection piece (61a) of the outer sheath (6) is connected in an airtight, watertight and germ-tight manner, and / or
- a second connection piece (61b) is integrally formed, and a teething ring (7b) is provided for a patient to be examined gastroscopically, which has an opening with a second receiving part (71b) to which the second connection piece (61b) of the outer sheath (6) is connected in an airtight, watertight and germ-tight manner, and / or
- a third connection piece (61c) is integrally formed, and a partition (7c) or cabin is provided for a patient to be examined to be positioned behind or in it, which has an opening with a third receiving part (71c) to which the third connection piece of the outer sheath (6) is connected in an airtight, watertight and germ-tight manner.

3. Endoscope according to Claim 1, **characterized in that** a film with an opening is welded to a distal end of the outer sheath (6) in an air-tight, water-tight and germ-tight manner and is stretched in front of a patient to be examined by means of a holding device.

4. Endoscope according to one of the preceding claims, **characterized in that** the inner sheath (3) and outer sheath (6)
- consist of airtight and watertight material that is impermeable to germs, and / or
- consist of flexibly compressible material, i.e. in particular can be rolled up or folded like a bellows.

5. Endoscope according to one of the Claims 1 to 4, **characterized in that**
- at least one instrument channel (10) runs in the shaft (1), which, on the shaft tip, opens into a distal instrument channel opening (101), and, on the handle (2), opens into a proximal instrument channel opening (102),
- the inner sleath (3) has a working hose opening (30c) which is congruent to the distal instrument channel opening (101) on the front side, and
- a working hose (4c) is integrally formed onto the working hose opening (30c) in a germ-tight manner, which is positioned in the instrumentation channel (10) and protrudes from the proximal instrumentation channel opening (102).

6. Endoscope according to Claim 5, **characterized in that** the working hose (4c) has a predetermined breaking point (4c0) in its section protruding from the proximal instrument channel opening (102), and that a protective hose (4c1) made of a polymer material, which has a higher elongation at break than a material of the working hose (4c), is attached to an outside or inside of the working hose (4c) in such a way that the protective hose (4c1) is connected in an air-, water- and germ-tight manner with the working hose (4c1) both in front of and behind the predetermined breaking point (4c0) .

7. Endoscope according to one of Claims 1 to 6, **characterized by**
- Valve holders (22a, 22b) in the handle (2), into which an air valve (5a) and a water valve (5b) or a combined air / water valve (5ab) integrated in a valve block (5ab) as well as a suction valve (5d) can be inserted, and
- a two-way hose distributor (24') with a distal, in particular conically shaped, end (241) inserted into a proximal end of the working hose (4c), the hose distributor (24') furthermore in particular having an instrument opening (242) and an annular nut (243) which is axially displaceable relative to the tube (4c) and by means of which it can be fastened to a proximal instrument channel opening (102) of the endoscope.

8. Endoscope according to one of Claims 1 to 6, **characterized by** a handle attachment (21) which is attached to the handle (2) and in which are provided:
- an air valve (5a) and a water valve (5b) in a valve holder (22),
- an instrument holder (23) with a 2-way hose distributor (24) inserted therein, at one distal end of which the working hose (4c) is connected,
- a suction valve (5d), and
- a suction hose (4d) which is connected to a first proximal end of the hose distributor (24) and the suction valve (5d).

9. Endoscope according to Claim 8, **characterized in that** an instrument protection (8) is connected to a second proximal end of the hose distributor (24), which consists of a bellows-like instrument hose (81) and which has an instrument protection cap (82) at each end with an opening at the front, and has a length which is greater than a length of the working hose (4c).

10. Endoscope according to one of the preceding claims, **characterized in that** the inner sheath (3) and/or the outer sheath (6) is attached to a proximal end of the shaft (1) on a outer surface of the shaft (1), in particular **in that** it is pressed by a clamp (91) or a rubber ring onto a proximal clamping bracket (93) which is arranged on the lateral surface of the shaft (1) and is underlaid with a proximal seal (92) and which clamping bracket has openings for air hose (4a) and water hose (4b).

11. Endoscope according to one of the preceding claims, **characterized in that** the distal end of the inner sheath (3) is designed as a substantially hollow cylindrical cap (31) with an open end face and, on an end face opposite the open end face, has an air hose opening (30a) and a waterhose opening (30b) to which the air hose (4a) and water hose (4b) are integrally formed, wherein
- a bracket-like clamp (33) with a click fastener (331, 331 ') is molded onto the cap (31) and the cap (31) is secured to the distal end of the shaft (1) by the fact that it sits on a distal clamping bracket (96) that is attached to the outer surface of the shaft (1) and underlaid with a distal seal (95) and has openings for the air hose (4a) and water hose (4b), or
- on an open end face of the cap (31) for clamping on the distal end of the shaft axially extending elastic branches are formed.

12. Endoscope according to one of the preceding claims, **characterized in that** the inner sheath (3) and outer sheath (6) are made in one piece, that is, the air, water and germ-tight connection of the outer sheath (6) with the proximal end of the inner sheath (3) is already made at production, for instance by welding, and/or the inner sheath (3) is underlaid with a fabric tube and/or the outer sheath (6) envelops the handle (2) and the handle attachment (21).

13. Endoscope according to Claims 8 and 12, **characterized in that** the two-way hose distributor (24) is integrated into the section of the outer sheath (6) which surrounds the handle (2) and the handle attachment (21).

14. Method for attaching a contamination protection device for an endoscope according to Claim 1, **characterized by** the steps:
- providing the optically transparent area at the distal end of the inner sheath (3) with a substance which makes optical contact with the optics (11) of the endoscope,
- attaching the handle attachment (21) to the handle (2),
- pnserting the distal end of the endoscope shaft (1) into the rolled-up inner sheath (3),
- condom-like unrolling of the inner sheath (3) enveloping the shaft (1) and the air hose (4a) and water hose (4b) positioned thereon,
- connecting the proximal ends of the air hose (4a) and water hose (4b) to the air valve (5a) and water valve (5b) on the handle attachment (21),
- sliding the rolled-up outer sheath (6) over the inner sheath (3) and attaching it to the proximal end of the inner sheath (3) in an air-, water- and germ-tight manner, and
- condom-like unrolling of the outer sheath (6) beyond the tip of the endoscope shaft (1)

15. Method for attaching a contamination protection device for an endoscope according to claim 2 according to the method steps according to claim 14, **characterized in that** subsequently the first or second or third connection piece (61a, b, c) of the outer sheath (6) is connected with the first or second or third receiving part (71a, b, c).

16. Method for attaching a contamination protection device for an endoscope according to claim 11 according to the method steps according to one of claims 14 or 15, **characterized in that** the following steps are carried out for fastening and connecting the inner sheath (3) and outer sheath (6):
- Gluing the proximal seal (92) onto the outer surface of the proximal end of the shaft (1),
- Attaching the proximal clamp (93) on the proximal seal (92),
- Gluing the distal seal (95) onto the lateral surface of the distal end of the shaft (1),
- Attaching the distal clamp (96) to the distal seal (95) using a positioning aid (960),
- Positioning the air hose (4a) and water hose (4b) in the feedthroughs on the distal clamping bracket (96),
- Sliding the cap (31) onto the distal clamping bracket (96),
- Positioning the air hose (4a) and water hose (4b) in the feedthroughs on the proximal clamping bracket (93),
- Positioning the proximal ends of the inner sheath (3) and outer sheath (6) over the proximal clamping bracket (93), and
- Clamping the inner sheath (3) and outer sheath (6) between the proximal clamping bracket (93) and the clamp (91) or the rubber ring.

17. Method for attaching a contamination protection device for an endoscope according to claim 11 according to the method steps according to one of claims 14 - 16, **characterized in that** before the introduction of the distal end of the shaft (1) into the rolled-up inner sheath (3), the proximal end of the working tube (4c) is inserted so far into the distal instrument channel opening (101) that it protrudes from the proximal instrument opening (102), and then
- the proximal end of the working hose (4c) is connected with the distal end of a 2-way hose distributor (24, 24 '),
- the first proximal end of the hose distributor (24, 24 ') is connected with the suction valve (5d) by means of the suction hose (4d), and
- the second proximal end of the hose distributor (24, 24 ') is connected with the bellows-like gathered instrument protection (8).

18. Method according to Claim 17, **characterized in that** the hose distributor (24') is fastened directly to the proximal instrument channel opening (102) by means of a ring nut (243).

19. Method for preparing an endoscope according to one of Claims 1 to 13 for a second contamination-protected endoscopic examination after the contamination-protected implementation of a first endoscopic examination, the last step of which is to pull the endoscope shaft out of the examined body opening,
**characterized by** the steps:
- Cutting through the outer sheath (6) in the area of its distal end and the air hose (4a) and water hose (4b) in the area of the handle (2) each with a curling iron, and
- Dismantling and disposing of the contamination protection device, with clamping brackets (93, 96) attached to the outer surface of the shaft possibly being left on the endoscope,
- Providing a further contamination protection comprising inner sheath (3), outer sheath (6) as well as water, air and work hoses (4a, 4b, 4c), and
- Assembling the further contamination protection according to one of Claims 14-18, the attachment of the proximal clamping bracket (93) and the distal clamping bracket (96) possibly being omitted as redundant.

## Revendications

1. Endoscope avec dispositif de protection contre la contamination comportant
- une manche rigide ou flexible (1), au bout de laquelle sont disposés une optique (11), un dispositif d'éclairage (12) et une tuyère (13),
- une poignée (2) fixée à la manche (1),
- un gaine intérieur (3) qui est fermé à l'une de ses extrémités distales et qui est optiquement transparent au moins sur la face avant et qui est enroulé le long de la manche (1) à la manière d'un préservatif,
- dans chaque cas au moins un tuyau d'air (4a) et un tuyau d'eau (4b), qui se terminent ouvertement à l'extrémité distale du gaine intérieur (3) et y sont reliés et qui sont placés entre l'arbre (1) et le gaine intérieure (3), et
dans lequel une gaine externe (6) envellope la gaine interne (3) et est reliée à celle-ci à une extrémité proximale de la gaine interne (3) d'une manière étanche à l'air, à l'eau et aux germes.

2. Endoscope selon la revendication 1, **caractérisé en ce qu'** à une extrémité distale de la gaine extérieure (6)
- un premier pièce de connexion (61a) est formé, et une paire de couches (7a) pour un patient à examiner par coloscopie est fournie, qui a une ouverture avec une première partie de réception (71a) à laquelle le premier connecteur (61a) de la gaine extérieure (6), est reliée de manière étanche à l'air, à l'eau et aux germes, et/ou
- une deuxième pièce de connexion (61b) est formée, et un anneau de dentition (7b) pour un patient à examiner par gastroscopie est prévu, qui présente une ouverture avec une deuxième partie de réception (71b) à laquelle la deuxième pièce de connexion (61b) de la gaine extérieure (6) est reliée de manière étanche à l'air, à l'eau et aux germes, et/ou
- une troisième pièce de connexion (61c) est formé, et il est prévu une cloison (7c) ou cabine pour un patient à examiner à positionner derrière ou dans elle, qui présente une ouverture avec une troisième partie de réception (71c) à laquelle la troisième pièce de connexion de la gaine extérieure (6) est connectée de manière étanche à l'air, à l'eau et aux germes.

3. Endoscope selon la revendication 1, **caractérisé en ce qu'**un film avec une ouverture est soudé à une extrémité distale de la gaine extérieure (6) de manière étanche à l'air, à l'eau et aux germes et est étiré devant un patient à examiner au moyen d'un dispositif de maintien.

4. Endoscope selon l'une des revendications précédentes, **caractérisé en ce que** la gaine intérieure (3) et la gaine extérieure (6)
- se composent d'un matériau étanche à l'air et à l'eau qui est imperméable aux germes, et/ou
- se composent d'un matériau flexible et compressible, c'est-à-dire qu'il peut notamment être enroulé ou plié comme un soufflet.

5. Endoscope selon l'une des revendications 1 à 4, **caractérisé en ce que**
- Au moins un canal d'instrument (10) s'étend dans la manche (1), qui s'ouvre dans une ouverture de canal d'instrument distal (101) sur la pointe de la manche et dans une ouverture de canal d'instrument proximal (102) sur la poignée (2),
- le gaine intérieur (3) présente une ouverture de tuyau de travail (30c) qui est congruente à l'ouverture de canal d'instrument distal (101) sur la face avant, et
- un tuyau de travail (4c) est formé sur l'ouverture de tuyau de travail (30c) de manière étanche aux germes, qui est positionné dans le canal d'instrumentation (10) et dépasse de l'ouverture de canal d'instrumentation proximale (102).

6. Endoscope selon la revendication 5, **caractérisé en ce que** le tuyau de travail (4c) présente un point de rupture prédéterminé (4c0) dans sa section dépassant de l'ouverture proximale du canal instrumental (102), et qu'un tuyau de protection (4c1) en matériau polymère, qui a un allongement à la rupture plus élevé qu'un matériau du tuyau de travail (4c) est fixé à l'extérieur ou à l'intérieur du tuyau de travail (4c) de telle sorte que le tuyau de protection (4c1) est connecté avec le tuyau de travail (4c1) d'une manière étanche à l'air, à l'eau et aux germes à la fois devant et derrière le point de rupture prédéterminé (4c0).

7. Endoscope selon l'une des revendications 1 à 6, **caractérisé par**
- une porte-valve (22a, 22b) dans la poignée (2), dans lesquels une valve à air (5a) et une valve à eau (5b) ou une valve combinée air/eau (5ab) intégrée dans un bloc de valve (5ab) et un la valve (5d) peut être insérée, et
- un distributeur de tuyau bidirectionnel (24') avec une extrémité distale, en particulier de forme conique, (241) insérée dans une extrémité proximale du tuyau de travail (4c), le distributeur de tuyau (24') ayant en outre en particulier une ouverture pour instrument (242) et possède un écrou annulaire (243) qui est déplaçable axialement par rapport au tuyau de travail (4c) et au moyen duquel il peut être fixé à une ouverture de canal d'instrument proximal (102) de l'endoscope.

8. Endoscope selon l'une des revendications 1 à 6, **caractérisé par** un embout de poignée (21) qui est fixé sur la poignée (2) et dans lequel :
- une vanne d'air (5a) et une vanne d'eau (5b) dans un porte-vanne (22),
- un support d'instrument (23) avec un distributeur de tuyau à 2 voies (24) inséré à l'intérieur, à une extrémité distale auquel le tuyau de travail (4c) est connecté,
- une soupape d'aspiration (5d), et
- un tuyau d'aspiration (4d) qui est connecté à une première extrémité proximale du distributeur de tuyau (24) et à la soupape d'aspiration (5d).

9. Endoscope selon la revendication 8, **caractérisé en ce qu' une** protection d'instrument (8) est reliée à une seconde extrémité proximale du distributeur de tuyau (24), qui se compose d'un tuyau d'instrument en forme de soufflet (81) qui a un capuchon de protection d'instrument (82 ) à chaque extrémité avec une ouverture à l'avant et a une longueur qui est supérieure à une longueur du tuyau de travail (4c).

10. Endoscope selon l'une des revendications précédentes, **caractérisé en ce que** la gaine interne (3) et/ou la gaine externe (6) est fixée à une extrémité proximale de la manche (1) sur une surface latérale de la manche (1), en particulier **en ce qu'**il est fixé par une pince (91) ou un anneau en caoutchouc pouvant être pressé sur un support de serrage proximal (93) qui est disposé sur la surface latérale de la manche (1) et est recouvert d'un joint proximal (92 ) et qui a des ouvertures pour le tuyau d'air (4a) et le tuyau d'eau (4b).

11. Endoscope selon l'une des revendications précédentes, **caractérisé en ce que** l'extrémité distale de la gaine intérieure (3) est conçue comme un capuchon cylindrique sensiblement creux (31) avec une face d'extrémité ouverte, qui présente une ouverture de tuyau d'air (30a) et un ouverture de tuyau d'air (30a) sur une face d'extrémité opposée à la face d'extrémité ouverte Possède une ouverture de tuyau d'eau (30b) sur laquelle sont formés le tuyau d'air (4a) et le tuyau d'eau (4b), dans lequel
- une bride en forme d'équerre (33) avec une fermeture à encliquetage (331, 331') est surmoulée sur le capuchon (31) et le capuchon (31) est fixé à l'extrémité distale de la manche (1) par le fait qu'il est fixé à un La surface circonférentielle de l'arbre (1) et recouverte d'un joint distal (95) repose sur le support de serrage distal (96) qui comporte des ouvertures pour le tuyau d'air (4a) et le tuyau d'eau (4b), ou
- sur une face d'extrémité ouverte du capuchon (31) pour le serrage sur l'extrémité distale de l'arbre, des branches élastiques s'étendant axialement sont formées.

12. Endoscope selon l'une des revendications précédentes, **caractérisé en ce que** la gaine intérieure (3) et la gaine extérieure (6) sont réalisées d'un seul tenant, c'est-à-dire la liaison étanche à l'air, à l'eau et aux germes de la gaine extérieure (6) avec l'extrémité proximale de la gaine intérieure (3) est déjà maintenue du côté de la production, par exemple par soudage, et/ou la gaine intérieure (3) est recouverte d'un tube en tissu et/ou la gaine extérieure (6) gaine la poignée (2) et la fixation de poignée (21).

13. Endoscope selon les revendications 8 et 12, **caractérisé en ce que** le distributeur de tuyaux à deux voies (24) est intégré dans la section de l'gaine extérieure (6) qui entoure la pièce de poignée (2) et la fixation de poignée (21).

14. Procédé de fixation d'un dispositif de protection contre la contamination pour un endoscope selon la revendication 1, **caractérisé par** les étapes :
- Exposition de la zone optiquement transparente à l'extrémité distale de la gaine intérieure (3) avec une substance qui établit un contact optique avec l'optique (11) de l'endoscope,
- Fixez la fixation de poignée (21) à la poignée (2),
- Insertion de l'extrémité distale de la manche d'endoscope (1) dans la gaine intérieure enroulée (3),
- déroulement en forme de préservatif de la gaine intérieure (3) comprenant la manche (1) et le tuyau d'air (4a) et le tuyau d'eau (4b) positionnés dessus,
- Connectez les extrémités proximales du tuyau d'air (4a) et du tuyau d'eau (4b) à la vanne d'air (5a) et à la vanne d'eau (5b) sur la fixation de la poignée (21),
- Glisser la couverture extérieure enroulée (6) sur la couverture intérieure (3) et la fixer à l'extrémité proximale de la couverture intérieure (3) et qui est imperméable à l'air, à l'eau et aux germes, et
- déroulement semblable à un préservatif de l'gaine extérieure (6) au-delà de l'extrémité de la manche de l'endoscope (1).

15. Procédé de fixation d'un dispositif de protection contre la contamination pour un endoscope selon la revendication 2 selon les étapes de procédé selon la revendication 14, **caractérisé en ce qu'**alors le premier ou le deuxième ou le troisième connecteur (61a, b, c) de la gaine externe (6) avec le premier, le deuxième ou le troisième connecteur (71a, b, c) est connecté.

16. Procédé de fixation d'un dispositif de protection contre la contamination pour un endoscope selon la revendication 11 selon les étapes de procédé selon l'une des revendications 14 ou 15, **caractérisé en ce que** les étapes suivantes sont exécutées pour fixer et relier la gaine interne (3) et la gaine externe (6) :
- Collage du joint proximal (92) sur la surface latérale de l'extrémité proximale de la manche (1),
- Fixation de la pince proximale (93) au joint proximal (92),
- Collage du joint distal (95) sur la surface latérale de l'extrémité distale de la manche (1),
- Fixation de la pince distale (96) au joint distal (95) à l'aide d'une aide au positionnement (960),
- Positionner le tuyau d'air (4a) et le tuyau d'eau (4b) dans les passages du support de serrage distal (96),
- Placer le capuchon (31) sur l'étrier de serrage distal (96),
- Positionner le tuyau d'air (4a) et le tuyau d'eau (4b) dans les passages du support de serrage proximal (93),
- Positionner les extrémités proximales de la gaine intérieure (3) et de la gaine extérieure (6) sur le support de serrage proximal (93), et
- Serrage de la gaine intérieure (3) et de la gaine extérieure (6) entre l'étrier de serrage proximal (93) et la pince (91) ou la bague en caoutchouc

17. Procédé de fixation d'un dispositif de protection contre la contamination pour un endoscope selon la revendication 11 selon les étapes de procédé selon l'une des revendications 14 à 16, **caractérisé en ce qu'**avant l'introduction de l'extrémité distale de la manche (1) dans l'intérieur de la gaine (3) enroulé, l'extrémité proximale du tuyau de travail (4c) est insérée si loin dans l'ouverture distale du canal d'instrument (101) qu'elle dépasse de l'ouverture proximale d'instrument (102), puis
- l'extrémité proximale du tuyau de travail (4c) est connecté avec l'extrémité distale d'un distributeur de tuyau à 2 voies (24, 24'),
- la première extrémité proximale du tuyau distributeur (24, 24') est connecté avec la soupape d'aspiration (5d) au moyen du tuyau d'aspiration (4d), et
- la deuxième extrémité proximale du tuyau distributeur (24, 24') est connecté avec la protection d'instrument froncée en forme de soufflet (8).

18. Procédé selon la revendication 17, **caractérisé en ce que** le tuyau distributeur (24') est fixé directement sur l'ouverture proximale du canal d'instruments (102) au moyen d'un écrou annulaire (243).

19. Procédé de préparation d'un endoscope selon l'une des revendications 1 à 13 pour un deuxième examen endoscopique protégé contre la contamination après la mise en œuvre protégée contre la contamination d'un premier examen endoscopique, dont la dernière étape consiste à retirer la manche de l'endoscope de l'ouverture corporelle examinée ,
**caractérisé par** les étapes :
- Découper la gaine extérieure (6) au niveau de son extrémité distale et le tuyau d'air (4a) et le tuyau d'eau (4b) au niveau du manche (2) chacun avec un fer à friser, et
- Démontage et élimination du dispositif de protection contre la contamination, avec étriers de serrage (93, 96) fixés sur la surface extérieure de la manche pouvant être laissés sur l'endoscope,
- Fourniture d'une protection supplémentaire contre la contamination comprenant une gaine intérieure (3), une gaine extérieure (6) ainsi que des tuyaux d'eau, d'air et de travail (4a, 4b, 4c), et
- Assemblage de la protection supplémentaire contre la contamination selon l'une des revendications 14 à 18, la fixation de l'étrier de serrage proximal (93) et de l'étrier de serrage distal (96) étant éventuellement omis car redondant.
